# EUROPEAN PATENT SPECIFICATION

(11) **EP 4 027 124 B1**
(45) Date of publication and mention of the grant of the patent: **28.01.2026**
(21) Application number: 20869633.6
(22) Date of filing: 25.09.2020
(51) Int. Cl.: G01K 13/20, G01J 5/12, G01J 5/00, G01J 5/02, G01J 5/06, A61B 5/01, A61B 5/00

(54) **HUMAN BODY TEMPERATURE MEASUREMENT METHOD AND WRIST WEARABLE DEVICE**
MESSVERFAHREN FÜR MENSCHLICHE KÖRPERTEMPERATUR UND AM HANDGELENK TRAGBARES GERÄT
PROCÉDÉ DE MESURE DE LA TEMPÉRATURE DU CORPS HUMAIN ET DIPOSITIF PORTABLE AU POIGNET

(30) Priority: 29.09.2019 CN 201910937161
(43) Date of publication of application: 13.07.2022
(73) Proprietor: Huawei Technologies Co., Ltd., Shenzhen, Guangdong 518129 (CN)
(72) Inventor: LI, Yue, Shenzhen, Guangdong 518129 (CN); YANG, Bin, Shenzhen, Guangdong 518129 (CN); REN, Huichao, Shenzhen, Guangdong 518129 (CN); LIU, Xiangyu, Shenzhen, Guangdong 518129 (CN)
(74) Representative: Pfenning, Meinig & Partner mbB
(86) International application number: PCT/CN2020/117574
(87) International publication number: WO 2021/057873

(56) References cited:
- WO-A1-2019/133469
- CN-A- 102 106 724
- CN-A- 105 310 659
- CN-A- 106 725 355
- CN-A- 106 725 355
- CN-A- 106 725 355
- CN-A- 109 419 496
- CN-A- 109 655 177
- CN-U- 203 299 558
- CN-Y- 2 634 511
- JP-A- 2012 237 670
- JP-A- 2012 237 670
- US-A1- 2012 319 847
- US-A1- 2016 113 517
- US-A1- 2016 273 967

## Description

### TECHNICAL FIELD

This application relates to the field of computers, and in particular, to a body temperature measurement method, an electronic device, and a computer-readable storage medium.

### BACKGROUND

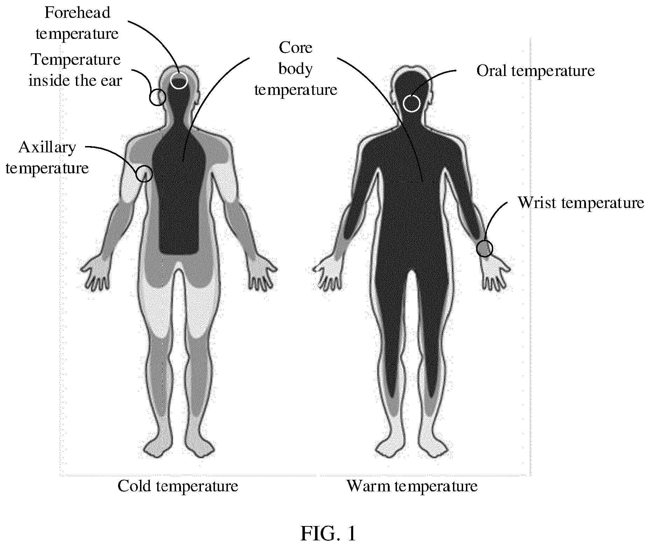

Clinically, a body temperature is a core body temperature. As shown in FIG. 1, the core body temperature (core body temperature, CBT), also referred to as a body temperature, is a temperature inside a chest cavity and an abdomen and of central nerves. As shown in FIG. 1, the body temperature differs slightly in cold and warm environments, is usually relatively constant, and fluctuates in a small range. The body temperature is used as one of four vital signs of a human body, and many physiological activities of the human body, such as hormone regulation, immune response, and natural rhythm are accompanied with changes in the body temperature. Therefore, body temperature measurement results, especially continuous measurement results, are of great significance for female physiological cycle management, biological rhythm regulation, chronic disease management, and other applications.

As shown in FIG. 1, the body temperature is the internal temperature of the human body and is not convenient to measure. A rectal temperature, an oral temperature (sublingual), and a tympanic membrane temperature (inside an ear) are closest to the body temperature, and an axillary temperature, a forehead temperature, and a chest temperature have relatively stable correspondences with the body temperature. Therefore, in an actual measurement scenario, temperature measurement is usually performed at a position, for example, an axilla, a hypoglossis (in an oral cavity), a tympanic membrane (inside an ear), an axilla, a forehead, or a chest, and a measured temperature is used as the body temperature. It is difficult for an existing body temperature measurement manner to balance measurement precision and convenience. Therefore, user experience is poor.
US 2016/273967 A1 discloses a wearable device with combined sensing capabilities, which includes a wearable assembly and at least one multi-function sensor module. The wearable assembly is suitable to be worn on a part of a user's body. The wearable assembly includes at least one light-transmissible window. The multi-function sensor module is located inside the wearable assembly, for performing an image sensing function and an infrared temperature sensing function. The multi-function sensor module includes an image sensor module for sensing a physical or a biological feature of an object through the light-transmissible window by way of image sensing; and an infrared temperature sensor module for sensing temperature through the light-transmissible window by way of infrared temperature sensing.
CN 106 725 355 A discloses a kind of worn type measurement of body temperature wrist strap and body temperature measurement method, a wrist temperature is measured using contact type temperature sensor and using infrared temperature sensor measuring an environment temperature is measured.
US 2016/113517 A1 concerns an electronic device for enhancing accuracy upon contactless body temperature measurement. The electronic device includes an image sensor for obtaining an image of an object, a temperature sensor disposed at a position adjacent to the image sensor for measuring a temperature of the obtained object, and a controller for performing control to determine the temperature of the object using a focal length of a camera module including the image sensor corresponding to a time of obtaining the image of the object and a temperature output from the temperature sensor corresponding to the time of obtaining the image of the object.
JP 2012 237670 A teaches a thermometer 1 including a first temperature sensor for detecting skin temperature of a user, a second temperature sensor 5 for detecting outside air temperature being an environmental temperature at which the user lives, and a control part for estimating the temperature of the user on the basis of the skin temperature detected by the first temperature sensor and the outside air temperature detected by the second temperature sensor, and a prescribed relation expression of preliminarily calculated body temperature, skin temperature and outside air temperature.

### SUMMARY

This invention provides a body temperature measurement method and a wrist wearable device, as defined in the independent claims, to improve comfort and convenience of a body temperature measurement process, and improve measurement precision.

According to a first aspect, this application provides a body temperature measurement method. The method is applied to a wrist wearable device, for example, a smart watch or a smart band.

In this embodiment of this application, a first temperature may be measured by using a first temperature sensor, and a second temperature may be measured by using a second temperature sensor, where a measurement moment of the first temperature is relatively close to that of the second temperature, and a measurement moment difference is within preset first duration. For example, measurement is performed at the same time or data is measured at the most recent time. The first temperature sensor is configured to measure a temperature at a forehead of a user, for example, a temperature sensor 11 disposed on a front surface of the smart watch. The second temperature sensor is configured to measure a temperature at a wrist of the user, for example, a temperature sensor 13 or a temperature sensor 14 disposed on a back surface of the smart watch. In this case, when a body temperature measurement result of the user is displayed on a display, a third temperature may be displayed. In this case, when the smart watch subsequently measures a fourth temperature by using the second temperature sensor, and the fourth temperature is the same as the second temperature, the third temperature may be displayed on the display. In this case, automatic measurement of the body temperature of the user can be implemented without a repeated measurement of the body temperature at the forehead of the user, and comfort is high. Moreover, because the temperature at the forehead is closer to a core body temperature, the third temperature is displayed after the second temperature is corrected by using the temperature at the forehead. This also helps improve measurement precision.

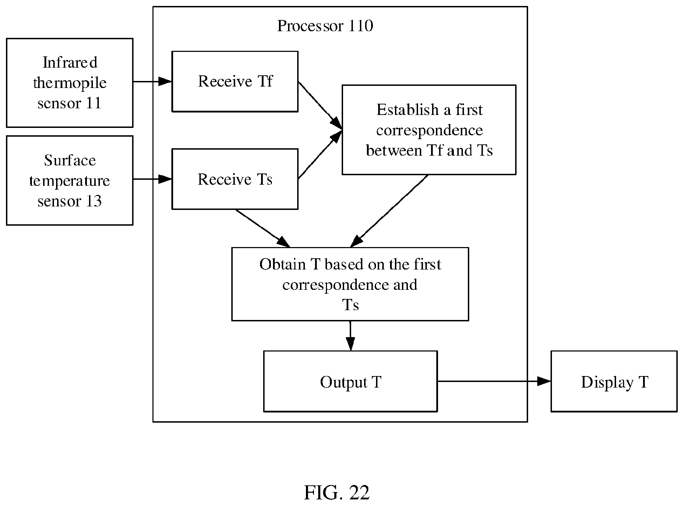

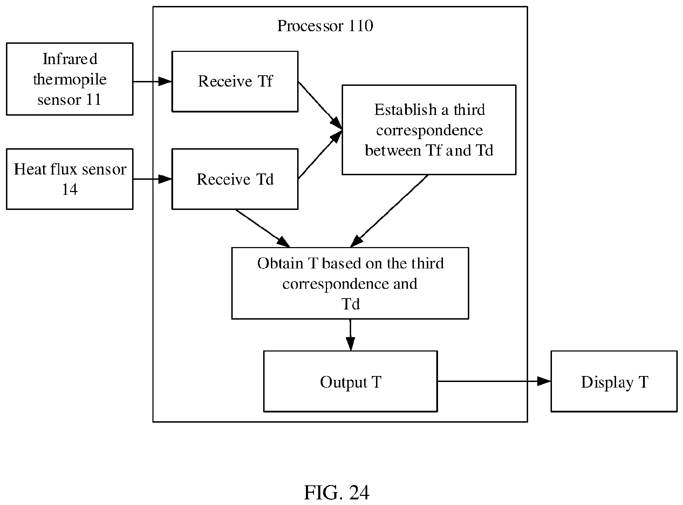

In an embodiment of this application, the third temperature may be the first temperature. For example, in the embodiment shown in FIG. 22, the first temperature is a forehead temperature Tf measured by the temperature sensor 11 and the second temperature is a skin surface temperature Ts measured by the temperature sensor 13, so that only a correspondence Tf=f(Ts) between Ts and Tf needs to be established. Then, when the temperature sensor 13 subsequently measures the same Ts value again, the Ts value may correspond to the same Tf, and the Tf value is displayed on the display. In another example, in the embodiment shown in FIG. 24, the first temperature is a forehead temperature Tf measured by the temperature sensor 11 and the second temperature is a deep tissue temperature Td measured by the temperature sensor 14, so that only a correspondence Tf=f(Td) between Td and Tf needs to be established. Then, when the temperature sensor 14 subsequently measures the same Td value again, the Td value may correspond to the same Tf, and the Tf value is displayed on the display.

In another embodiment of this application, the third temperature may alternatively be associated with the first temperature and the second temperature. The embodiments shown in FIG. 22 and FIG. 24 are still used as examples. In the embodiment shown in FIG. 22, the third temperature may be obtained by querying a table. For example, a correspondence table among Tf, Ts, and T is queried to obtain the third temperature (T) corresponding to the first temperature (Tf) and the second temperature (Ts in this case). Alternatively, the third temperature may be obtained by performing weighted calculation on Tf and Ts, for example, T=w1*Tf+w2*Ts. In another embodiment, as shown in FIG. 24, the third temperature may be obtained by querying a table. For example, a correspondence table among Tf, Td, and T is queried to obtain the third temperature (T) corresponding to the first temperature (Tf) and the second temperature (Td in this case). Alternatively, the third temperature may be obtained by performing weighted calculation on Tf and Td, for example, T=w5*Tf+w6*Td.

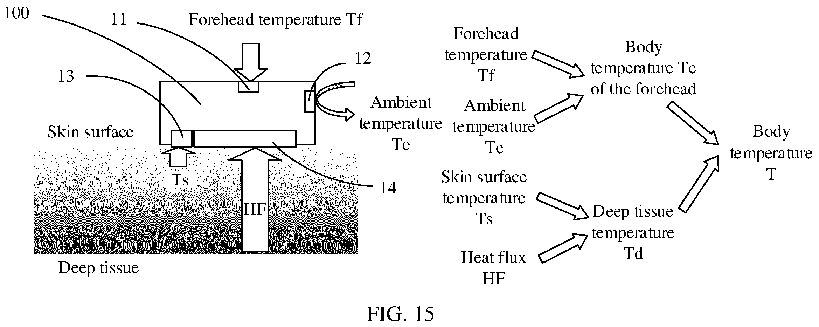

In addition, in this embodiment of this application, impact of an ambient temperature on a measured temperature may be further considered, so that the measured temperature is corrected by using the ambient temperature. In this case, the third temperature is associated with a fifth temperature, and the fifth temperature is obtained after the first temperature is corrected by using the ambient temperature. For example, as shown in FIG. 15, FIG. 21, FIG. 23, FIG. 25, and FIG. 26, the fifth temperature may be denoted as a body temperature Tc on the forehead, and Tc is obtained after the forehead temperature Tf is corrected by using the ambient temperature Te.

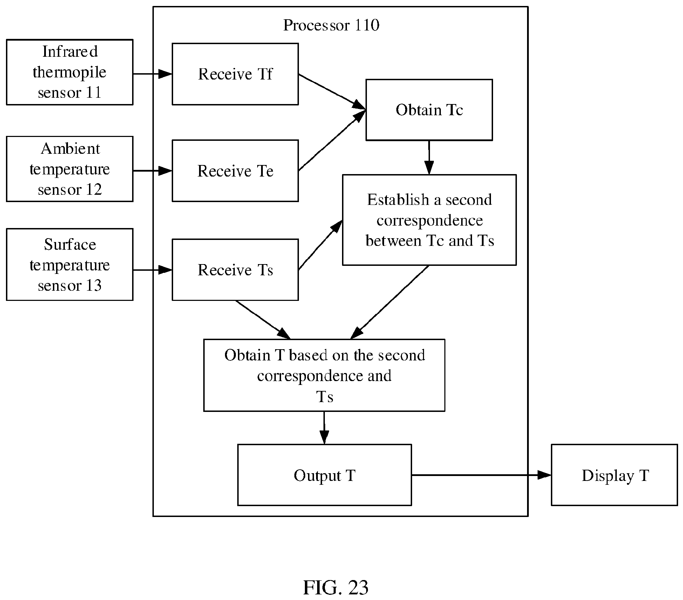

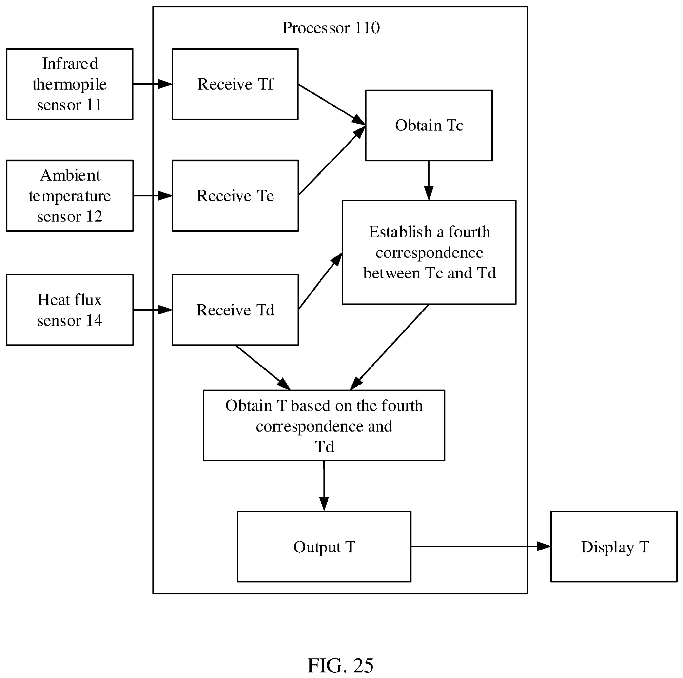

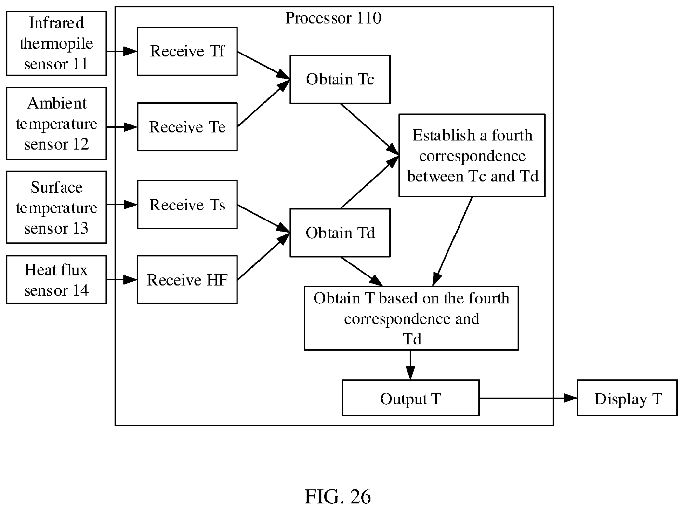

In this case, the third temperature may be the fifth temperature. That is, a value of Tc is displayed on the display. For example, in the embodiment shown in FIG. 23, Tc is obtained after the forehead temperature Tf is corrected by using the ambient temperature Te. In this way, a correspondence Tc=f(Ts) between Ts and Tc is established. Then, when the temperature sensor 13 subsequently measures the same Ts value again, the Ts value may correspond to the same Tc, and the Tc value is displayed on the display. In another example, in another embodiment, as shown in FIG. 25 or FIG. 26, Tc is obtained after the forehead temperature Tf is corrected by using the ambient temperature Te. In this way, a correspondence Tc=f(Td) between Td and Tc is established. Then, when the second temperature sensor subsequently measures the same Td value again, the Td value may correspond to the same Td, and the Tc value is displayed on the display.

In the foregoing embodiment, the ambient temperature may be measured by using a third temperature sensor. In this case, the third temperature sensor may be disposed in the smart watch, or may be disposed in another electronic device. Alternatively, the ambient temperature may be obtained by using a network method. For example, when the smart watch is connected to a mobile phone by using Bluetooth, the ambient temperature recorded in the mobile phone may be received by using Bluetooth.

As described above, in this embodiment of this application, the second temperature sensor may be configured to measure the deep tissue temperature at the wrist of the user. In this case, the second temperature sensor may be a heat flux sensor 14.

Alternatively, in another embodiment, the second temperature sensor is configured to measure the skin surface temperature at the wrist of the user. In this case, the second temperature sensor may be a surface temperature sensor 13.

In this case, an embodiment of this application further provides an implementation. Reference may be made to the embodiment shown in FIG. 26. In this embodiment, a first heat flux may alternatively be measured by using the heat flux sensor 14, where the heat flux sensor is configured to measure a heat flux between the skin surface temperature and the deep tissue temperature, and a difference between measurement moments of the first heat flux and the second temperature is within preset second duration. In other words, when the user actively measures the body temperature at the forehead, the heat flux sensor 14 may also measure a heat flux value. In this way, the heat flux value participates in calculation together with the second temperature (the skin surface temperature Ts measured by the temperature sensor 13 in this case), so that the body temperature of the user is obtained by using a mapping relationship between Tc and Td (may alternatively be obtained by using a mapping relationship between Tf and Td in another embodiment). Therefore, in this embodiment, when the body temperature of the user is automatically measured by using the wrist wearable device subsequently, a second heat flux may further be measured by using the heat flux sensor in addition to measuring the fourth temperature by using the second sensor, and a difference between measurement moments of the second heat flux and the fourth temperature is within the second duration. In this way, the third temperature is displayed on the display when the fourth temperature is the same as the second temperature and the first heat flux is the same as the second heat flux.

In addition, in this embodiment of this application, when the wrist wearable device automatically measures the body temperature of the user, if the measured fourth temperature is different from the second temperature, a correspondence between the second temperature and the first temperature may be obtained. Therefore, a sixth temperature corresponding to the fourth temperature is obtained based on the correspondence, and the sixth temperature is displayed on the display.

In this embodiment, the correspondence may be stored at a preset storage location. The correspondence may be preset by a developer in advance in the smart watch, or may be established by the smart watch. Therefore, in a possible embodiment, if the correspondence is established by the smart watch, data accumulated in a plurality of measurements may be used for establishing the correspondence. That is, the correspondence is established by using a plurality of first temperatures and a plurality of second temperatures; and the correspondence is stored at the preset storage location.

A threshold may be further preset, and the correspondence is established after a quantity of the first temperatures reaches the preset threshold. That is, after a particular amount of data is accumulated, the correspondence is established based on the data. This helps improve accuracy of the correspondence, and further, helps improve accuracy of the body temperature of the user that is finally displayed on the display. For example, in the embodiment shown in FIG. 22, when a quantity of times of active measurement of the body temperature by the user reaches K1, for example, 20, a mapping function Tf=f(Ts) may be established by using Tf acquired by an infrared thermopile sensor 11 in the 20 measurement processes and Ts corresponding to Tf.

After the correspondence is established, the smart watch may further update and maintain the correspondence. In an embodiment, after the correspondence is established, the correspondence is updated when the quantity of the received first temperatures reaches a preset quantity threshold. For example, in the embodiment shown in FIG. 22, after the mapping function is established, each time the user actively measures the body temperature for M1 times, for example, 30 times, the mapping function between Tf and Ts is redetermined by using the 30 pieces of Tf data and Ts data corresponding to Tf, to update the mapping function. In another example, in the embodiment shown in FIG. 24, when a quantity of times of active measurement by the user reaches M3, the processor of the smart watch may further update the mapping function between Tf and Td by using measurement data stored at the preset storage location, so that the third correspondence is closer to a recent body temperature status of the user. This helps improve measurement precision of the body temperature T.

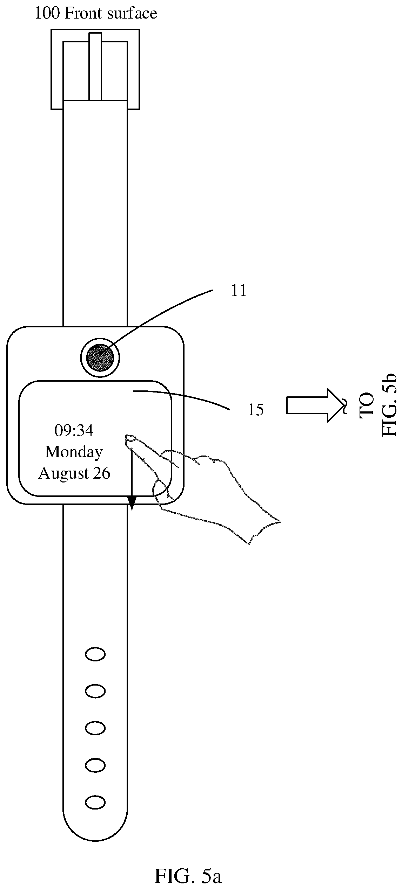

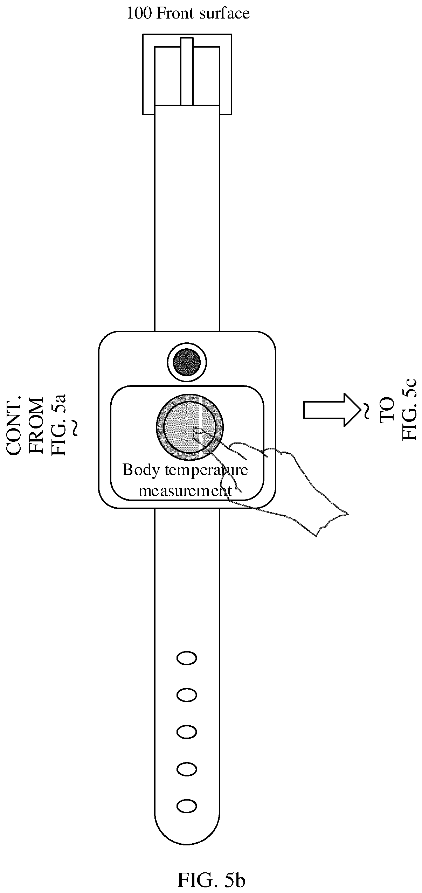

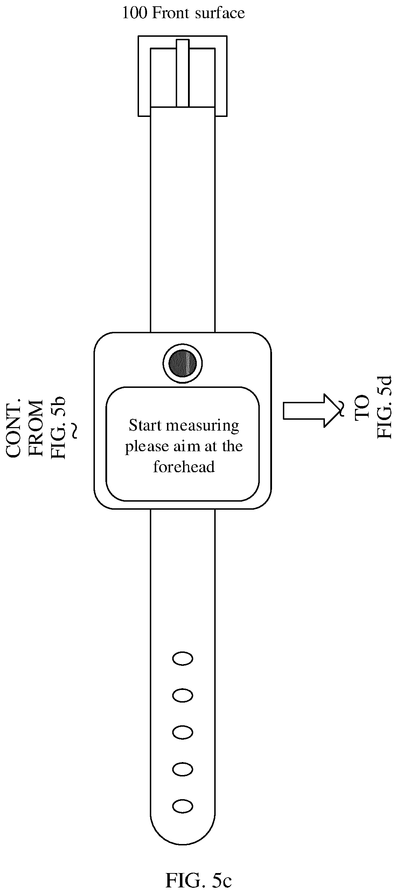

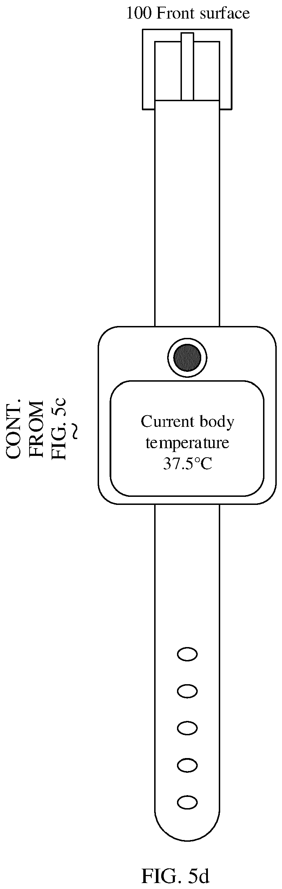

In this embodiment of this application, the first temperature sensor is configured to measure the forehead temperature of the user, and the first temperature sensor may measure the first temperature in response to receiving a body temperature measurement instruction triggered by the user, and output the first temperature. For example, in the embodiment shown in FIG. 5a, FIG. 5b, FIG. 5c, and FIG. 5d, the temperature sensor 11 may start to work after the user taps a virtual button of "body temperature measurement". In this way, the forehead temperature can be measured when the user raises the wrist to enable the temperature sensor 11 to be aligned with the forehead of the user.

The second temperature sensor measures temperature data and outputs the temperature data based on a preset period or moment. That is, the second temperature sensor may automatically measure the temperature, or a function of automatically measuring the body temperature of the user may be implemented in this way. In addition, in another embodiment, the second temperature sensor measures the second temperature when the first temperature sensor measures the first temperature. In this way, it can be ensured that the first temperature is close to the second temperature. During specific implementation, the first temperature sensor 11 may notify, in a manner of direct or indirect communication, the second temperature sensor to work. Alternatively, the second temperature sensor also starts to work in response to receiving the body temperature measurement instruction triggered by the user.

In this embodiment of this application, the first temperature sensor is disposed in the wrist wearable device, and is not in contact with the skin of the wrist of the user; and/or, the second temperature sensor is disposed in the wrist wearable device, and is in contact with the skin of the wrist of the user. For example, in the embodiments shown in FIG. 5a, FIG. 5b, FIG. 5c, and FIG. 5d to FIG. 7, the temperature sensor 11 is disposed on a front surface of the smart watch. In another embodiment, in the embodiment shown in FIG. 10, the temperature sensor 13 is disposed on a back surface of the smart watch. In the embodiments shown in FIG. 12 and FIG. 13, the temperature sensor 14 is disposed on the back surface of the smart watch. In another example, in the embodiment shown in FIG. 14, the temperature sensor 11 is disposed on the front surface of the smart watch, and the temperature sensor 13 and the temperature sensor 14 are disposed on the back surface of the smart watch.

In addition, the display 15 may alternatively be disposed on the wrist wearable device, or may be a display of a terminal device, and the terminal device is communicatively connected to the wrist wearable device.

According to a second aspect, an embodiment of this application provides a wrist wearable device, including:
one or more processors, one or more memories, and one or more computer programs, where the one or more computer programs are stored in the one or more memories, the one or more computer programs include instructions, and when the instructions are executed by the wrist wearable device, the electronic device is enabled to perform the method in any embodiment in the first aspect.

In addition, the electronic device further includes the following one or more temperature sensors: a first temperature sensor, configured to measure a temperature at a forehead of a user; and a second temperature sensor, configured to measure a temperature at a wrist of the user.

In another embodiment, the electronic device further includes: a third temperature sensor, configured to measure an ambient temperature.

The second temperature sensor is configured to measure a deep tissue temperature at the wrist of the user, or is configured to measure a skin deep temperature at the wrist of the user. Then, when the second temperature sensor is configured to measure the skin surface temperature at the wrist of the user, the electronic device further includes: a heat flux sensor, configured to measure a heat flux between the skin surface temperature and the deep tissue temperature.

In addition, the electronic device further includes: a display, configured to display temperature data.

In this embodiment of this application, the electronic device is a wrist wearable device, for example, a smart watch or a smart band.

According to a third aspect, a non-claimed embodiment of this application further provides a computer storage medium, including computer instructions. When the computer instructions are run on an electronic device, the electronic device is enabled to perform the method in any one of the foregoing implementations.

According to a fourth aspect, a non-claimed embodiment of this application further provides a computer program product. When the computer program product is run on an electronic device, the electronic device is enabled to perform the method in any one of the foregoing implementations.

In conclusion, the body temperature measurement method, the electronic device, and the computer-readable storage medium that are provided in this application can improve comfort and convenience of a body temperature measurement process and improve measurement precision.

### BRIEF DESCRIPTION OF DRAWINGS

FIG. 1 is a schematic diagram of body temperatures according to an embodiment of this application;

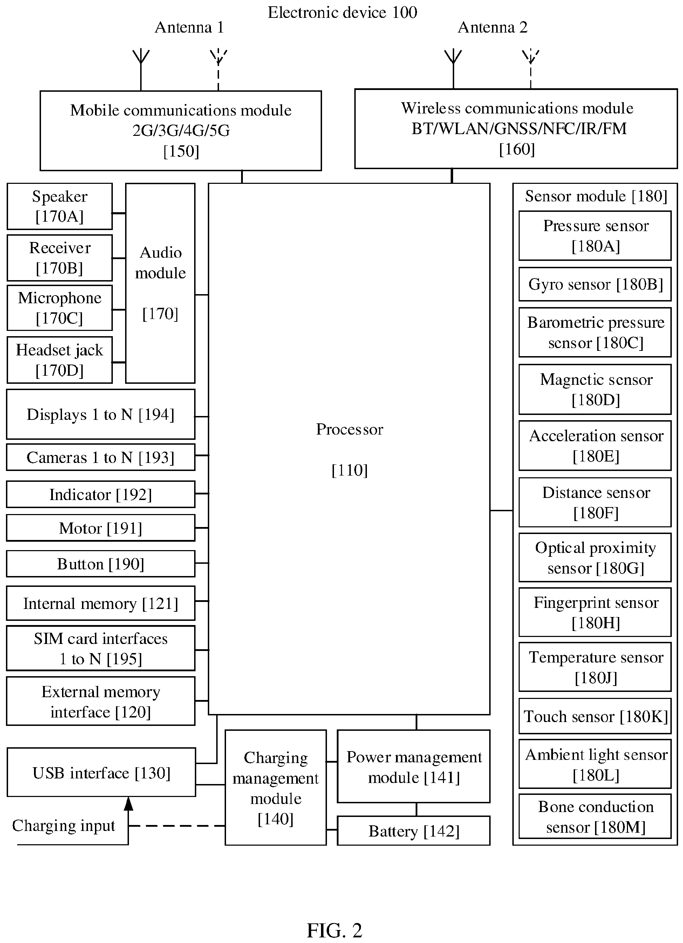

FIG. 2 is a schematic diagram of a structure of an electronic device according to an embodiment of this application;

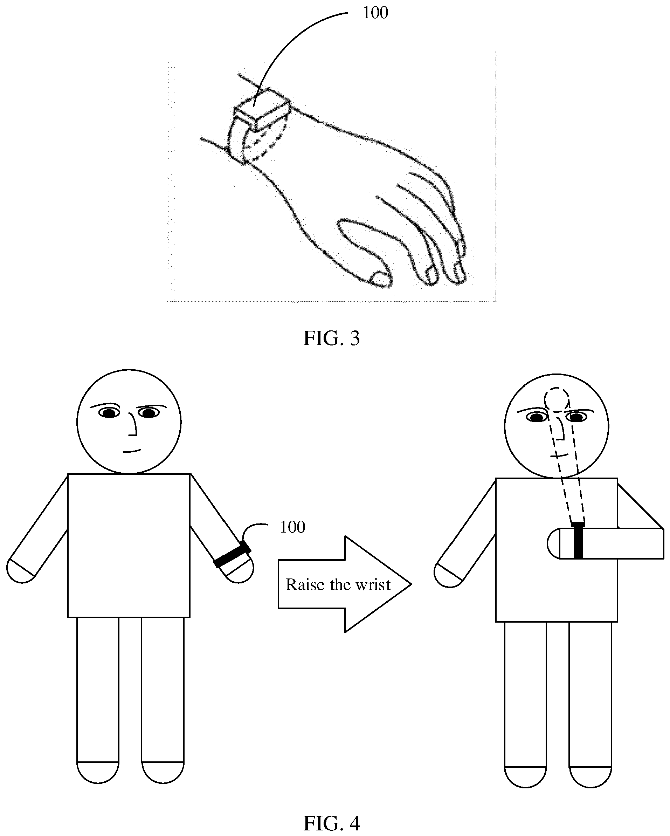

FIG. 3 is a schematic diagram of a body temperature measurement scenario according to an embodiment of this application;
FIG. 4 is a schematic diagram of a body temperature measurement method according to an embodiment of this application;
FIG. 5a, FIG. 5b, FIG. 5c, and FIG. 5d are a schematic diagram of another body temperature measurement method according to an embodiment of this application;

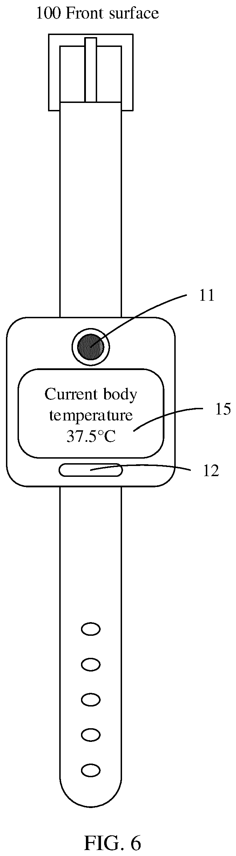

FIG. 6 is another schematic diagram of a structure of an electronic device according to an embodiment of this application;

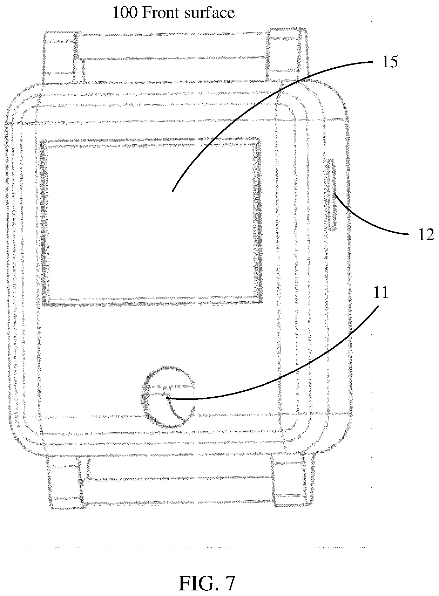

FIG. 7 is another schematic diagram of a structure of an electronic device according to an embodiment of this application;

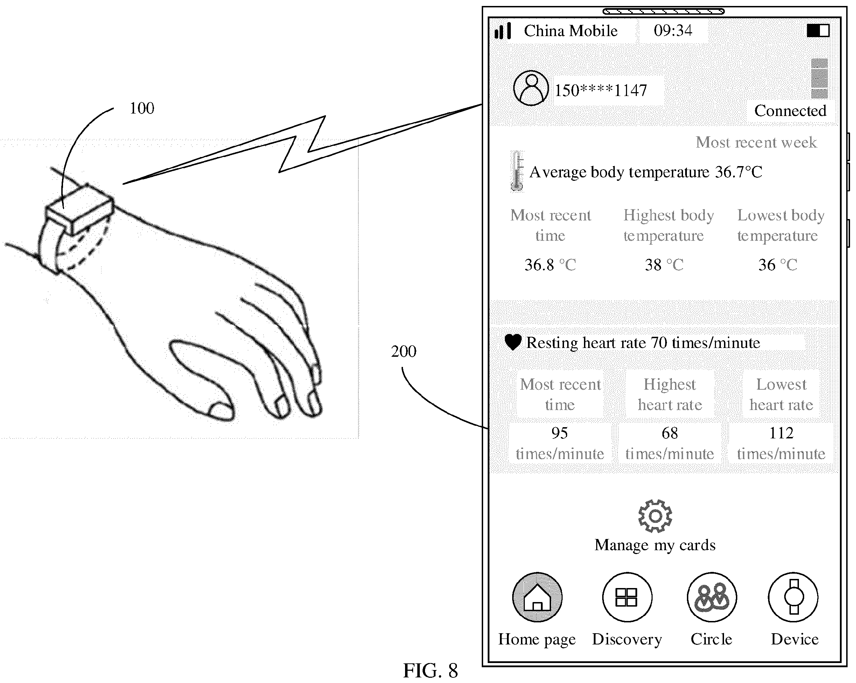

FIG. 8 is a schematic diagram of another body temperature measurement scenario according to an embodiment of this application;

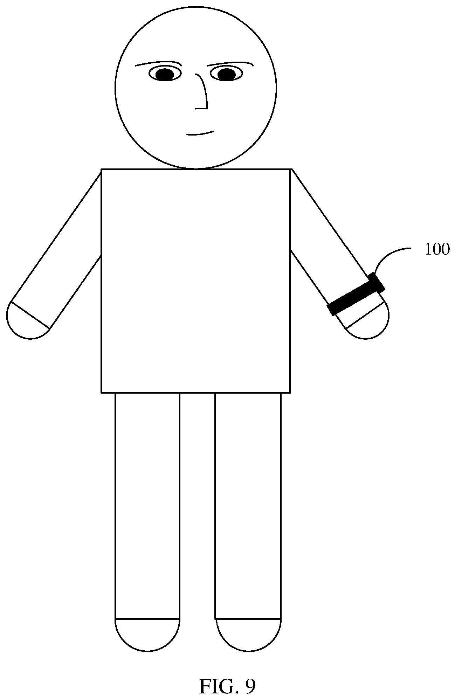

FIG. 9 is a schematic diagram of another body temperature measurement scenario according to an embodiment of this application;

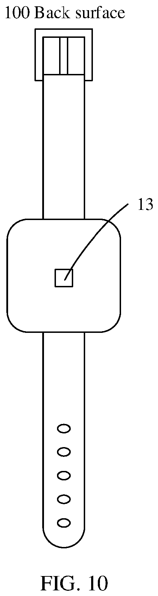

FIG. 10 is another schematic diagram of a structure of an electronic device according to an embodiment of this application;

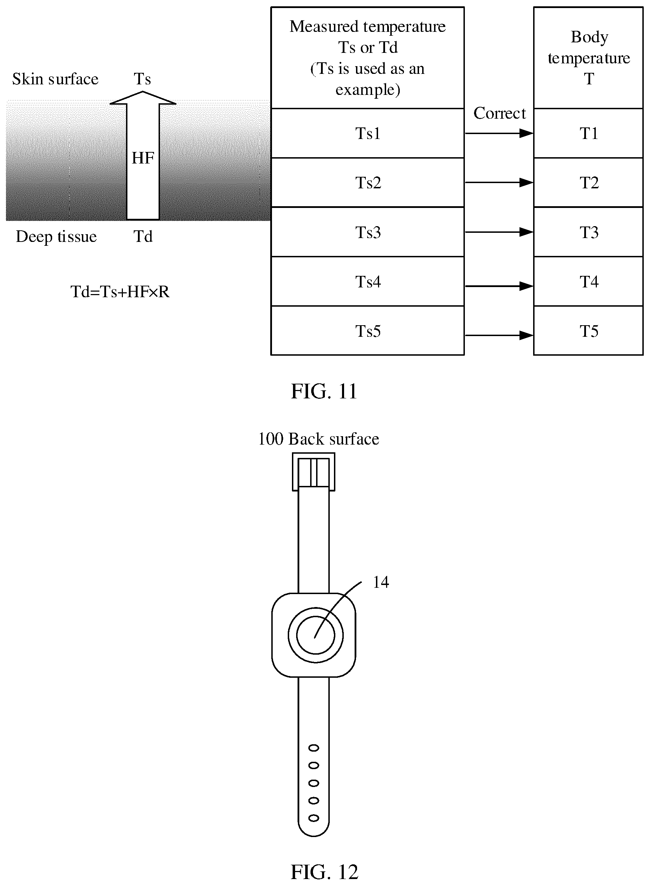

FIG. 11 is a schematic diagram of a body temperature measurement principle according to an embodiment of this application;
FIG. 12 is another schematic diagram of a structure of an electronic device according to an embodiment of this application;

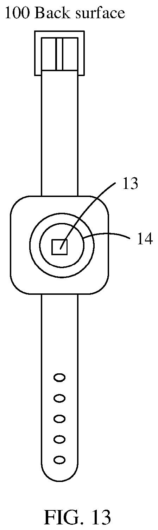

FIG. 13 is another schematic diagram of a structure of an electronic device according to an embodiment of this application;

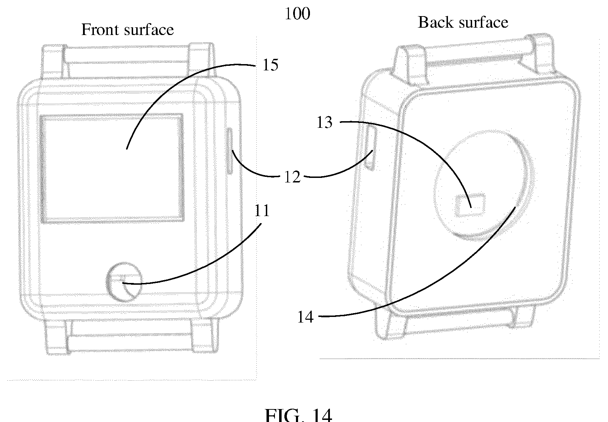

FIG. 14 is another schematic diagram of a structure of an electronic device according to an embodiment of this application;
FIG. 15 is a schematic diagram of another body temperature measurement principle according to an embodiment of this application;

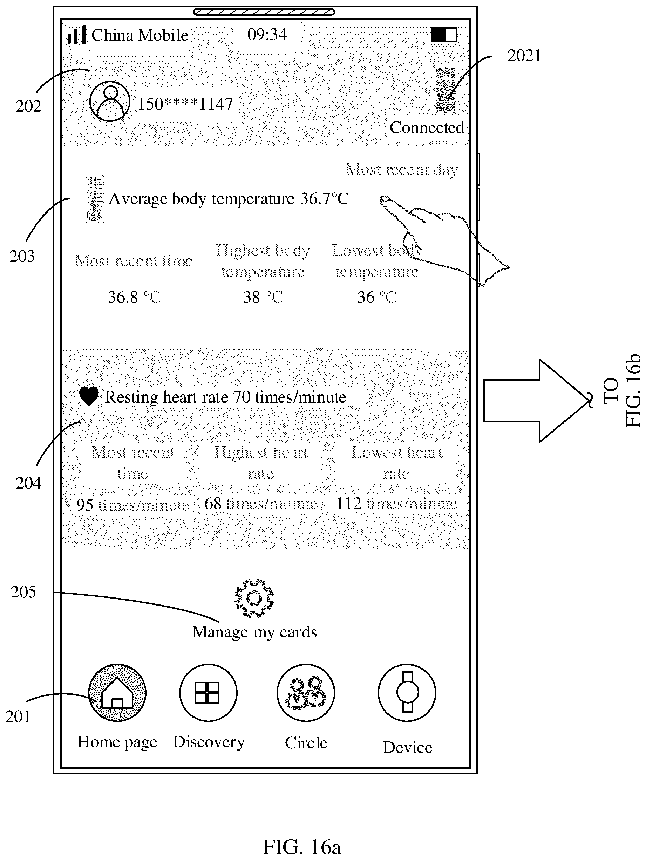

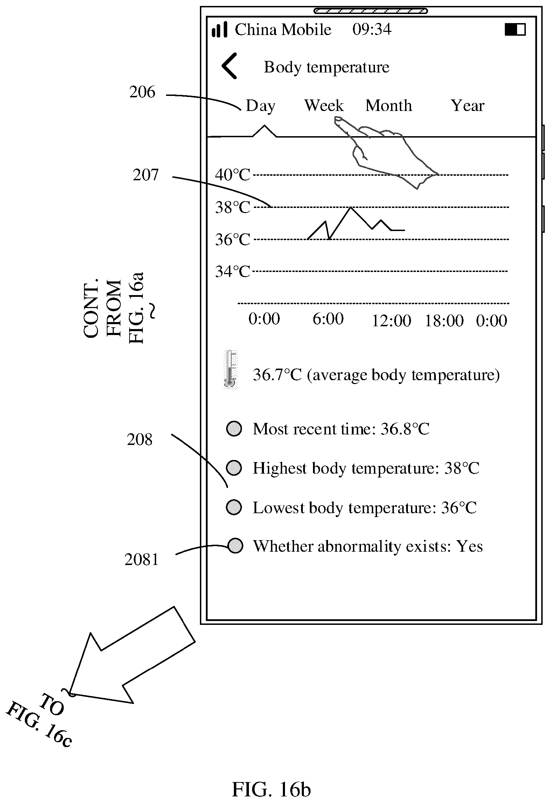

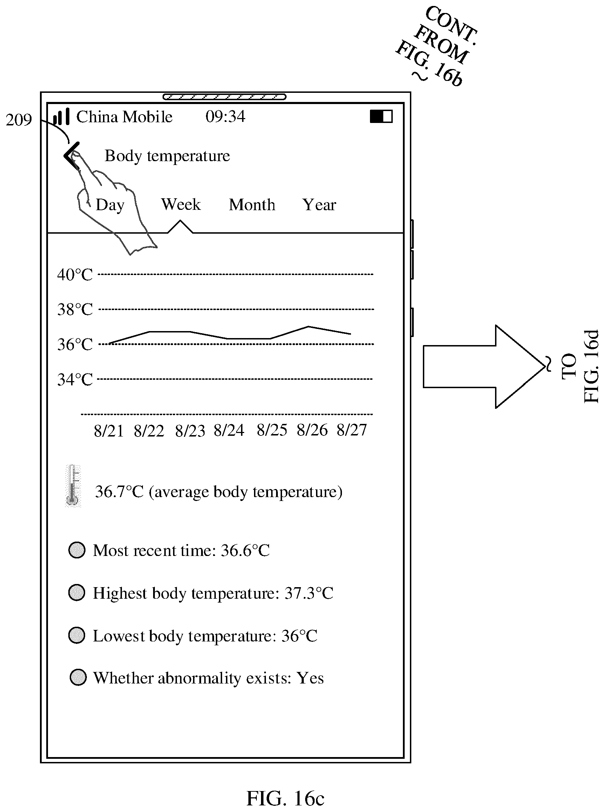

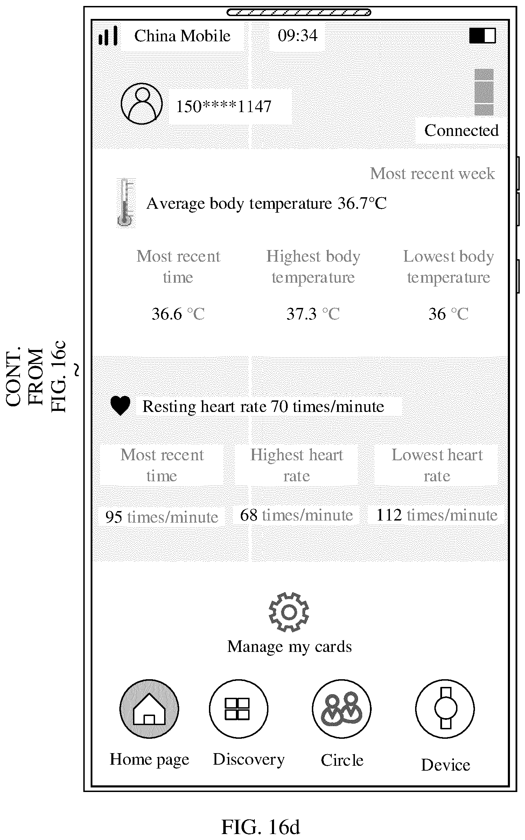

FIG. 16a, FIG. 16b, FIG. 16c, and FIG. 16d are a schematic diagram of a human-computer interaction interface according to an embodiment of this application;

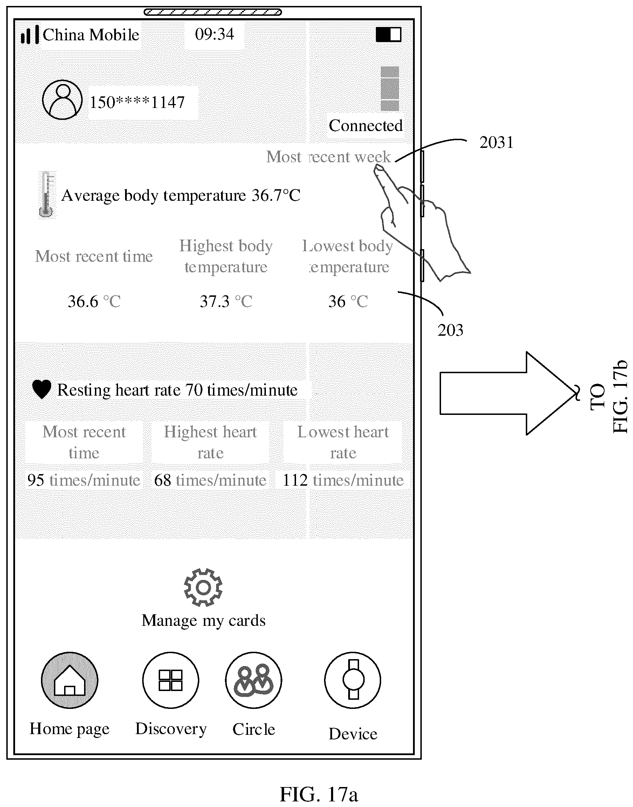

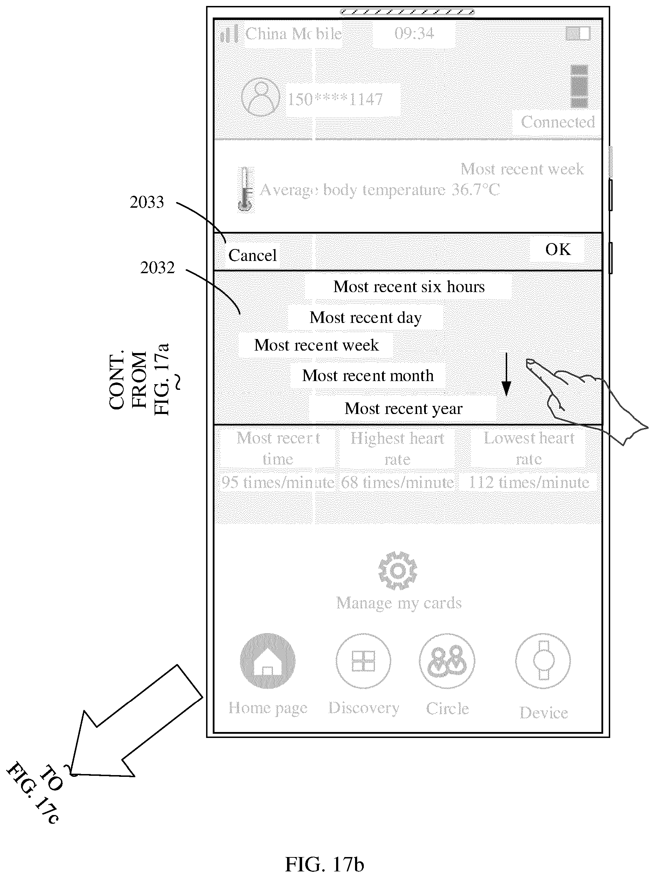

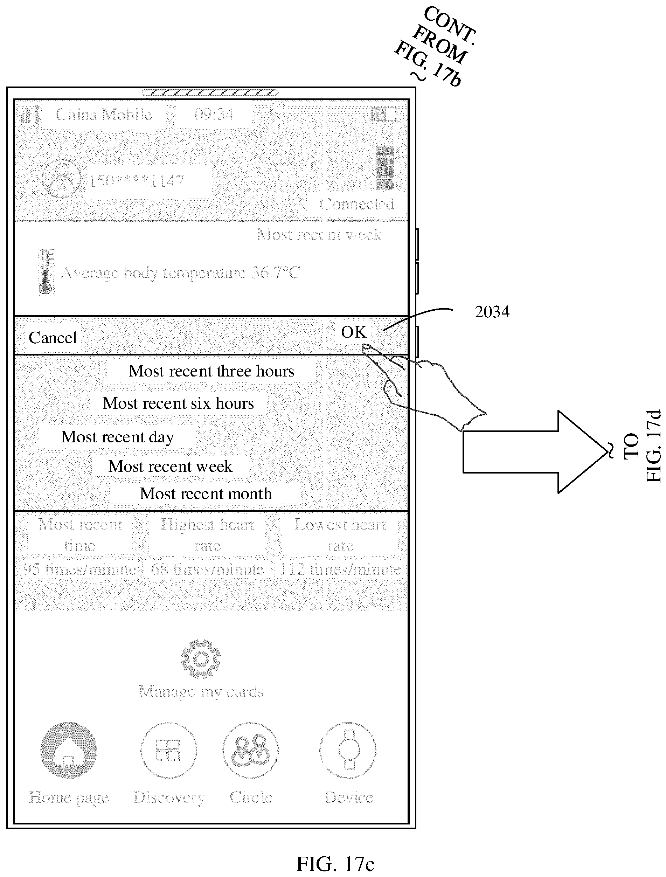

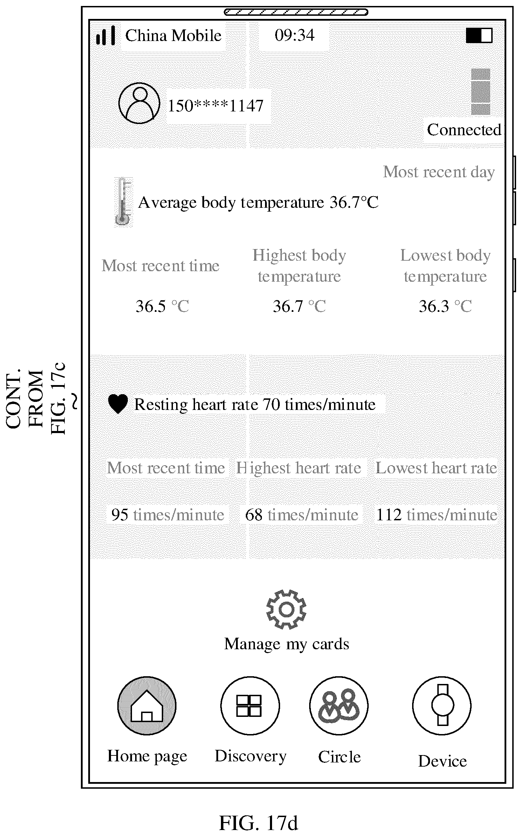

FIG. 17a, FIG. 17b, FIG. 17c, and FIG. 17d are a schematic diagram of another human-computer interaction interface according to an embodiment of this application;

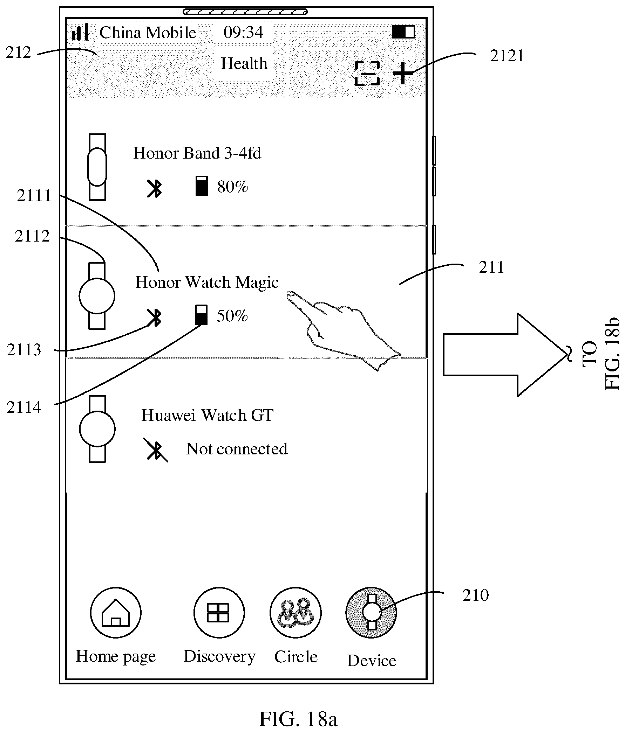

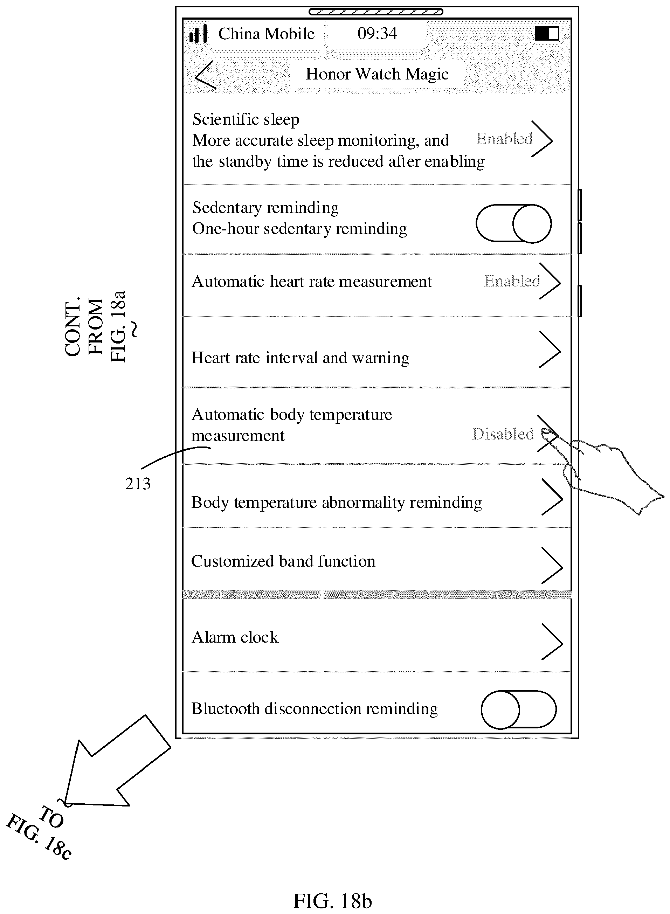

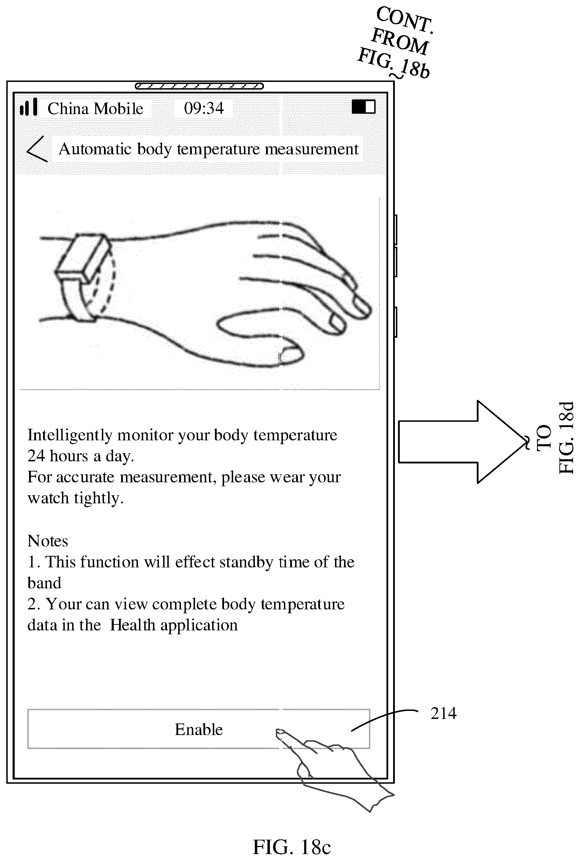

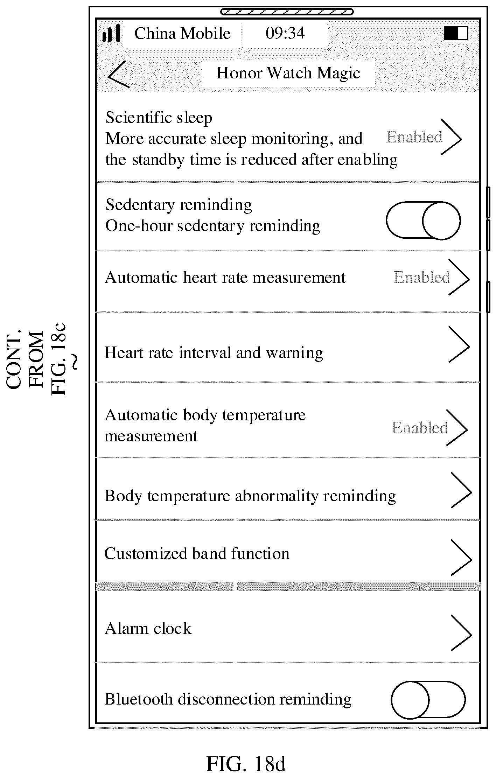

FIG. 18a, FIG. 18b, FIG. 18c, and FIG. 18d are a schematic diagram of another human-computer interaction interface according to an embodiment of this application;

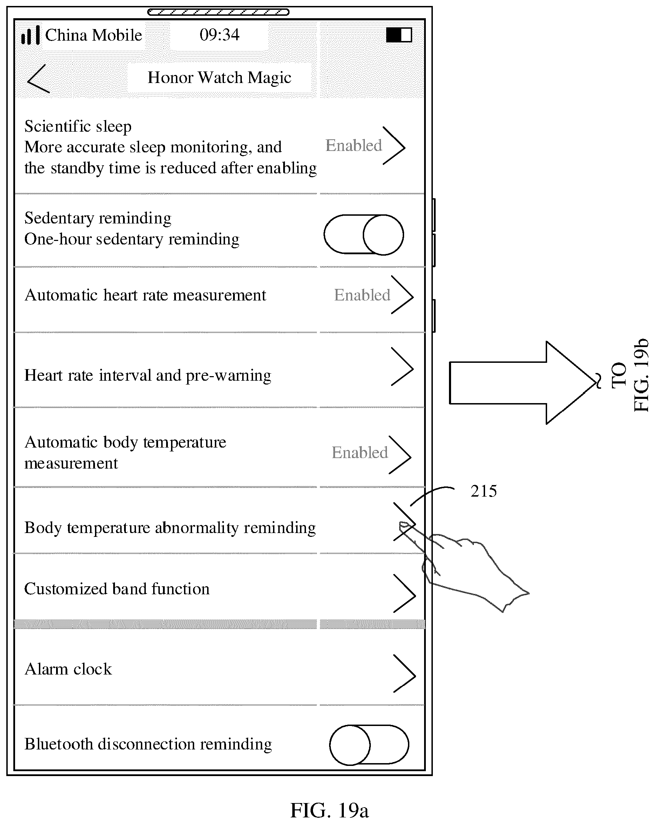

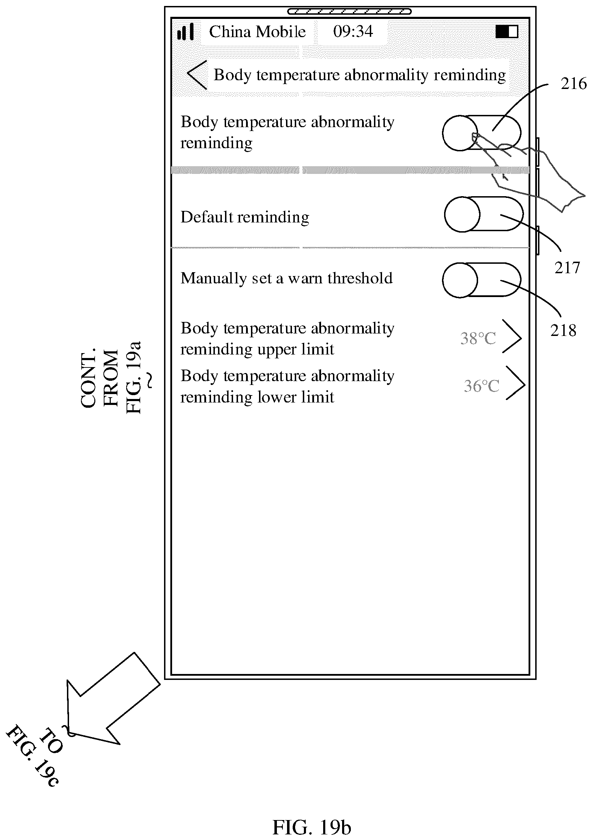

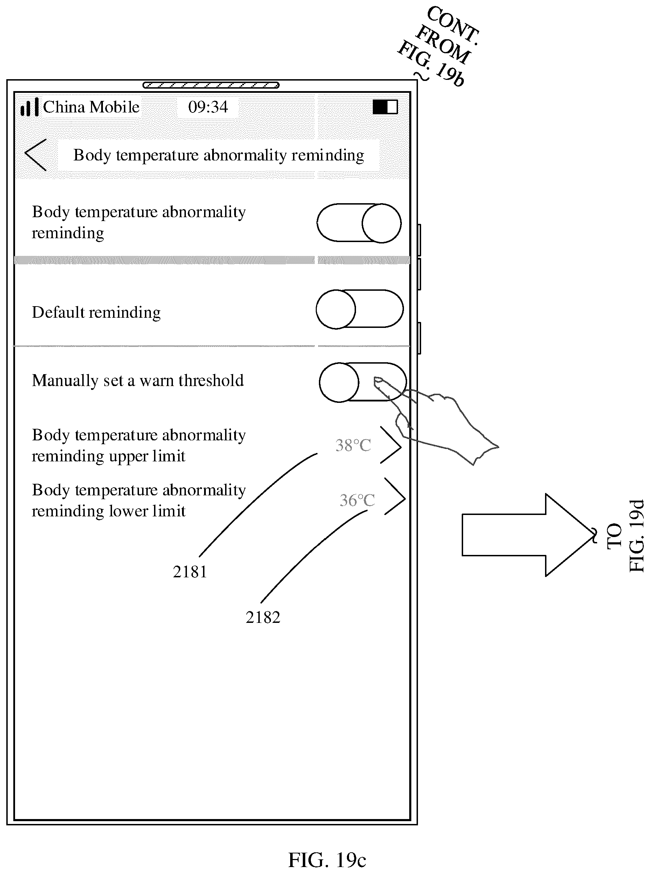

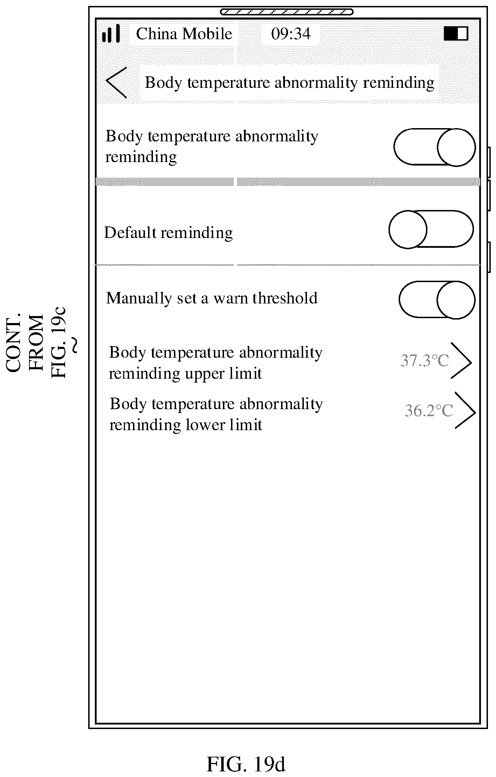

FIG. 19a, FIG. 19b, FIG. 19c, and FIG. 19d are a schematic diagram of another human-computer interaction interface according to an embodiment of this application;

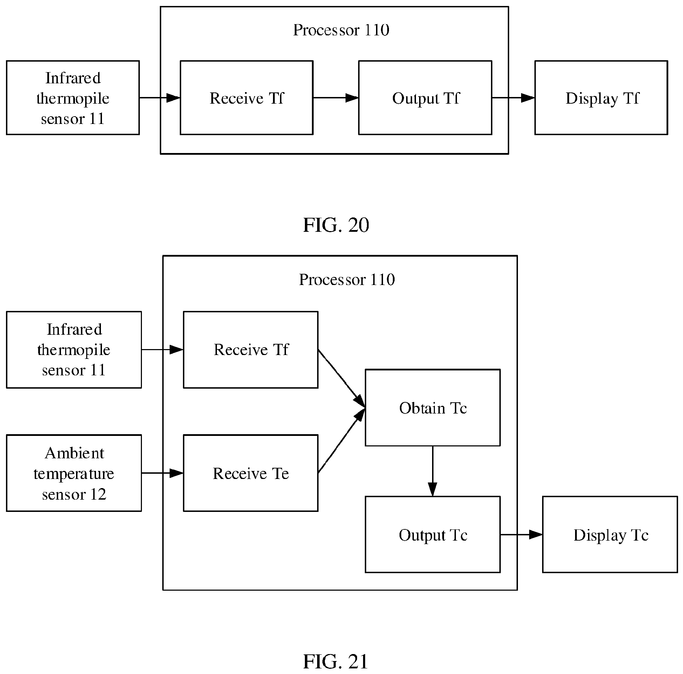

FIG. 20 is a schematic diagram of another body temperature measurement method according to a non-claimed embodiment of this application;
FIG. 21 is a schematic diagram of another body temperature measurement method according to a non-claimed embodiment of this application;
FIG. 22 is a schematic diagram of another body temperature measurement method according to an embodiment of this application;
FIG. 23 is a schematic diagram of another body temperature measurement method according to an embodiment of this application;
FIG. 24 is a schematic diagram of another body temperature measurement method according to an embodiment of this application;
FIG. 25 is a schematic diagram of another body temperature measurement method according to an embodiment of this application;
FIG. 26 is a schematic diagram of another body temperature measurement method according to an embodiment of this application;

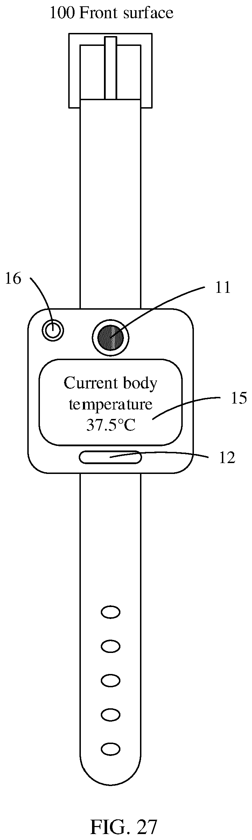

FIG. 27 is another schematic diagram of a structure of an electronic device according to an embodiment of this application;

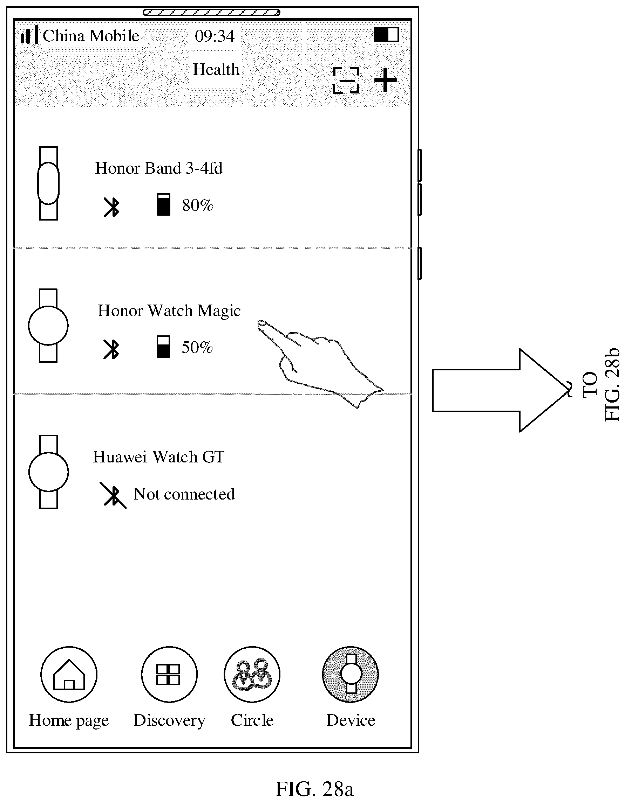

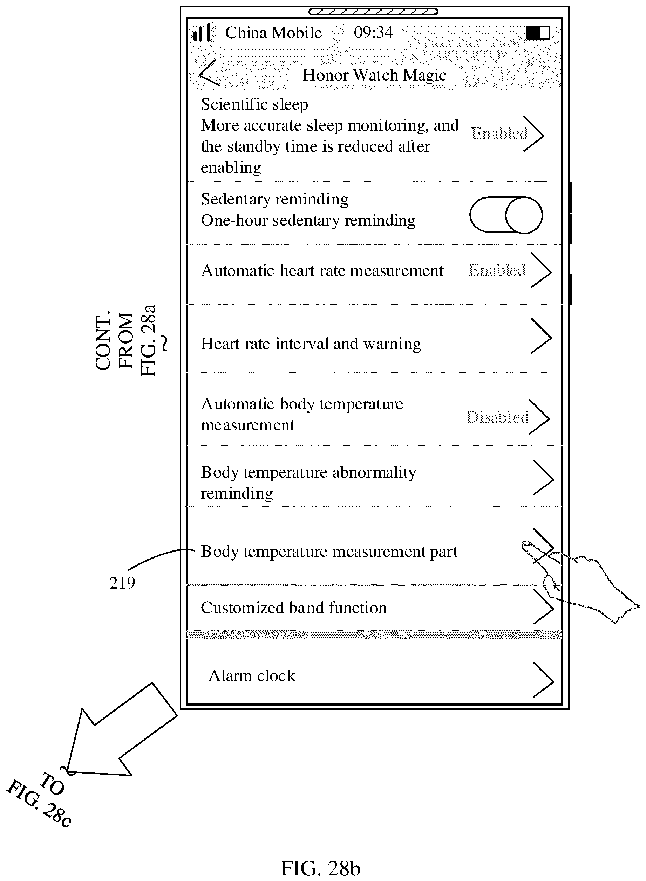

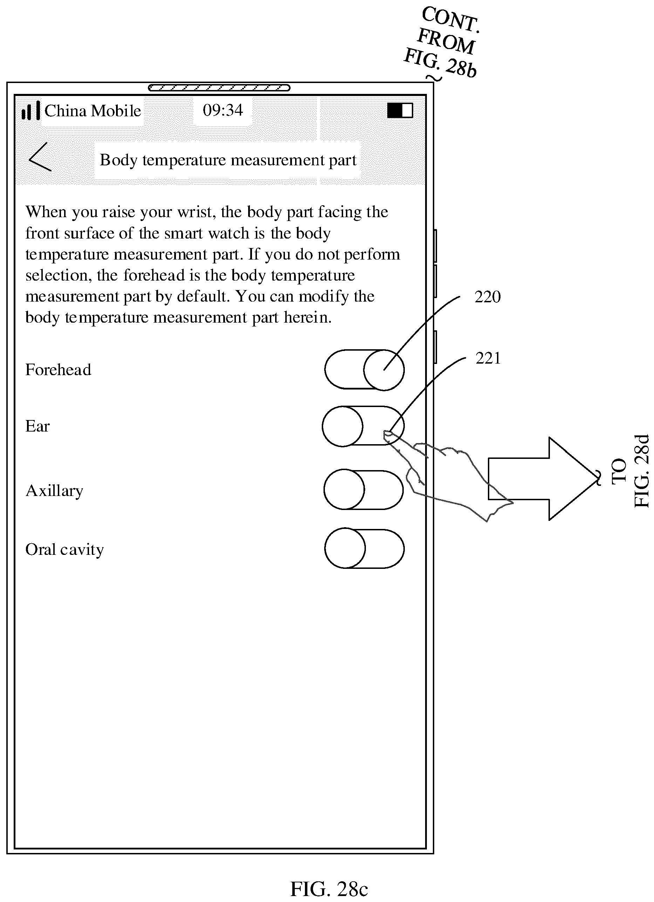

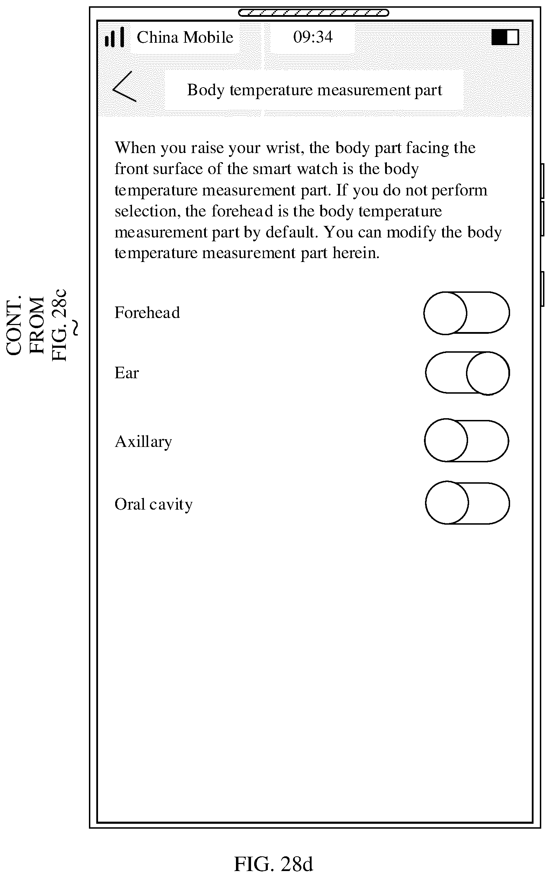

FIG. 28a, FIG. 28b, FIG. 28c, and FIG. 28d are a schematic diagram of another human-computer interaction interface according to an embodiment of this application;

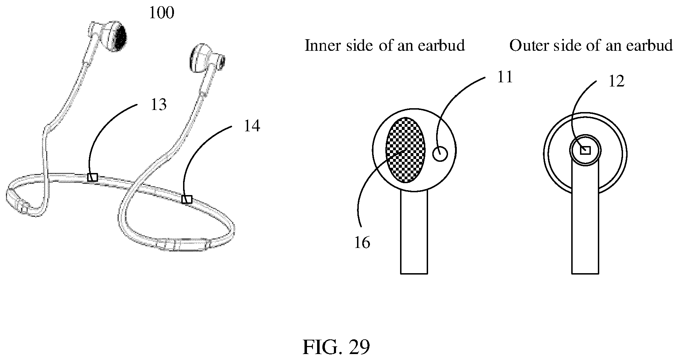

FIG. 29 is another schematic diagram of a structure of an electronic device according to a non-claimedembodiment of this application; and

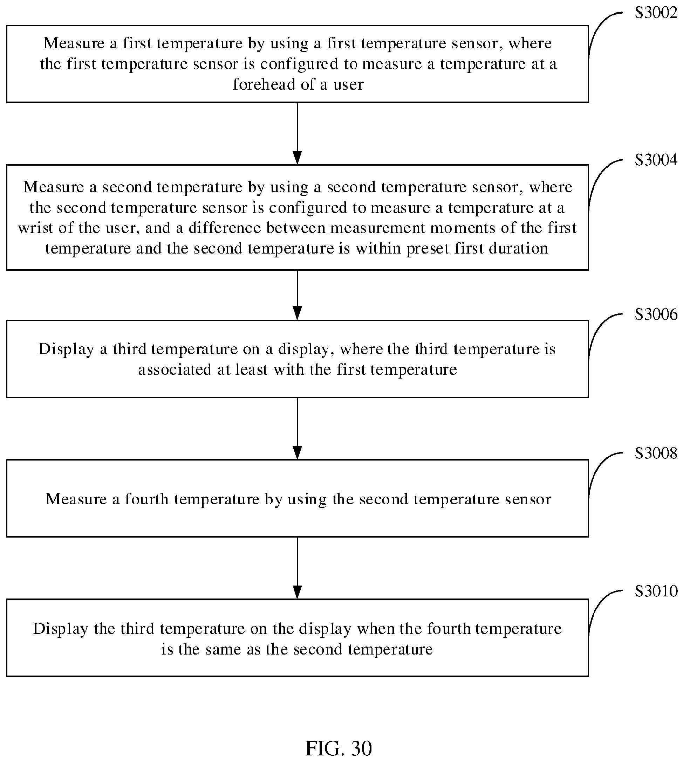

FIG. 30 is a schematic diagram of another body temperature measurement method according to an embodiment of this application.

### DESCRIPTION OF EMBODIMENTS

Implementations of embodiments are described in detail below with reference to the accompanying drawings. In the descriptions of embodiments of this application, unless otherwise specified, "/" means "or". For example, A/B may represent A or B. In this specification, "and/or" describes only an association between associated objects and represents that three relationships may exist. For example, A and/or B may represent the following three cases: Only A exists, both A and B exist, and only B exists. In addition, in the descriptions of embodiments of this application, "a plurality of" means two or more.

The technical solutions provided in this application may be applied to any electronic device. For example, FIG. 2 is a schematic diagram of a structure of an electronic device.

The electronic device may include a processor 110, an external memory interface 120, an internal memory 121, a universal serial bus (universal serial bus, USB) interface 130, a charging management module 140, a power management module 141, a battery 142, an antenna 1, an antenna 2, a mobile communications module 150, a wireless communications module 160, an audio module 170, a speaker 170A, a receiver 170B, a microphone 170C, a headset jack 170D, a sensor 180, a button 190, a motor 191, an indicator 192, a camera 193, a display 194, a subscriber identification module (subscriber identification module, SIM) card interface 195, and the like. It can be understood that the structure shown in embodiments does not constitute a specific limitation on the electronic device.

In some other embodiments of this application, the electronic device may include more or fewer components than those shown in the figure, or some components may be combined, or some components may be split, or different component arrangements may be used. For example, when the electronic device is a smart watch or a smart band, the smart watch does not need to be provided with one or more of the SIM card interface 195, the camera 193, the button 190, the receiver 170B, the microphone 170C, the headset jack 170D, the external memory interface 120, and the USB interface 130. In another example, when the electronic device is a smart headset, the smart headset does not need to be provided with one or more of the SIM card interface 195, the camera 193, the display 194, the receiver 170B, the microphone 170C, the headset jack 170D, the external memory interface 120, the USB interface 130, or some sensors in the sensor module 180 (for example, a gyro sensor 180B, a barometric pressure sensor 180C, a magnetic sensor 180D, an acceleration sensor 180E, a distance sensor 180F, and a fingerprint sensor 180H). The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent devices, or may be integrated into one or more processors. In some embodiments, the electronic device may also include one or more processors 110. The controller may be a nerve center and a command center of the electronic device. The controller may generate an operation control signal based on instruction operation code and a time sequence signal, to complete control of instruction fetching and instruction execution. A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor may directly invoke the instructions or the data from the memory. This avoids repeated access and reduces a waiting time of the processor 110, thereby improving efficiency of the electronic device.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like. The USB interface 130 is an interface that conforms to a USB standard specification, and may be specifically a mini USB interface, a micro USB interface, a USB Type-C interface, or the like. The USB interface 130 may be configured to connect to a charger to charge the electronic device, or may be configured to transmit data between the electronic device and a peripheral device, or may be configured to connect to a headset and play audio through the headset.

It may be understood that an interface connection relationship between the modules that is shown in embodiments of the present invention is merely an example for description, and does not constitute a limitation on the structure of the electronic device. In some other embodiments of this application, the electronic device may alternatively use an interface connection manner different from that in the foregoing embodiment, or a combination of a plurality of interface connection manners.

The charging management module 140 is configured to receive a charging input from the charger. The charger may be a wireless charger or a wired charger. In some embodiments of wired charging, the charging management module 140 may receive a charging input from the wired charger through the USB interface 130. In some embodiments of wireless charging, the charging management module 140 may receive a wireless charging input through a wireless charging coil of the electronic device. The charging management module 140 may further supply power to the electronic device through the power management module 141 while charging the battery 142.

The power management module 141 is configured to connect the battery 142 and the charging management module 140 to the processor 110. The power management module 141 receives an input from the battery 142 and/or the charging management module 140, and supplies power to the processor 110, the internal memory 121, the display 194, the camera 193, the wireless communications module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device may be implemented through the antenna 1, the antenna 2, the mobile communications module 150, the wireless communications module 160, the modem processor, the baseband processor, and the like. The antenna 1 and the antenna 2 are configured to transmit and receive electromagnetic wave signals. Each antenna of the electronic device may be configured to cover one or more communication frequency bands. Different antennas may be further multiplexed, to improve antenna utilization. For example, the antenna 1 may be multiplexed as a diversity antenna in a wireless local area network. In some other embodiments, an antenna may be used in combination with a tuning switch.

The mobile communications module 150 may provide a wireless communication solution that includes 2G/3G/4G/5G and the like and that is applied to the electronic device. The mobile communications module 150 may include at least one filter, a switch, a power amplifier, a low noise amplifier, and the like. The mobile communications module 150 may receive an electromagnetic wave through the antenna 1, perform processing such as filtering and amplification on the received electromagnetic wave, and transmit a processed electromagnetic wave to a modem processor for demodulation. The mobile communications module 150 may further amplify a signal modulated by the modem processor, and convert an amplified signal into an electromagnetic wave for radiation through the antenna 1. In some embodiments, at least some function modules of the mobile communication module 150 may be disposed in the processor 110. In some embodiments, at least some function modules of the mobile communications module 150 may be disposed in a same device as at least some modules of the processor 110.

The modem processor may include a modulator and a demodulator. The modulator is configured to modulate a to-be-sent low-frequency baseband signal into a medium-high-frequency signal. The demodulator is configured to demodulate a received electromagnetic wave signal into a low-frequency baseband signal. Then, the demodulator transmits the low-frequency baseband signal obtained through demodulation to the baseband processor for processing. After being processed by the baseband processor, the low-frequency baseband signal is transmitted to the application processor. The application processor outputs a sound signal through an audio device (which is not limited to the speaker 170A, the receiver 170B, or the like), or displays an image or a video on the display 194. In some embodiments, the modem processor may be an independent component. In some other embodiments, the modem processor may be independent of the processor 110, and is disposed in a same device as the mobile communications module 150 or another function module.

The wireless communications module 160 may provide a wireless communication solution that is applied to the electronic device and that includes a wireless local area network (wireless local area network, WLAN), Bluetooth, a global navigation satellite system (global navigation satellite system, GNSS), frequency modulation (frequency modulation, FM), NFC, an infrared (infrared, IR) technology, or the like. The wireless communications module 160 may be one or more components integrating at least one communications processor module. The wireless communications module 160 receives an electromagnetic wave through the antenna 2, performs frequency modulation and filtering processing on an electromagnetic wave signal, and sends a processed signal to the processor 110. The wireless communications module 160 may further receive a to-be-sent signal from the processor 110, perform frequency modulation and amplification on the signal, and convert a processed signal into an electromagnetic wave for radiation through the antenna 2.

In some embodiments, in the electronic device, the antenna 1 is coupled to the mobile communications module 150, and the antenna 2 is coupled to the wireless communications module 160, so that the electronic device can communicate with a network and another device by using a wireless communications technology. The wireless communications technology may include GSM, GPRS, CDMA, WCDMA, TD-SCDMA, LTE, GNSS, WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device implements a display function through the GPU, the display 194, the application processor, and the like. The GPU is a microprocessor for image processing, and is connected to the display 194 and the application processor. The GPU is configured to: perform mathematical and geometric calculation, and render an image. The processor 110 may include one or more GPUs, which execute instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or more displays 194.

The electronic device may implement a photographing function through the ISP, one or more cameras 193, the video codec, the GPU, one or more displays 194, the application processor, and the like.

The ISP is configured to process data fed back by the camera 193. For example, during photographing, a shutter is pressed, light is transmitted to a photosensitive element of the camera through a lens, an optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected to the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format such as RGB or YUV. In some embodiments, the electronic device 100 may include one or more cameras 193.

The digital signal processor is configured to process a digital signal, and may process another digital signal in addition to the digital image signal. For example, when the electronic device 100 selects a frequency, the digital signal processor is configured to perform Fourier transform and the like on frequency energy.

The video codec is configured to compress or decompress a digital video. The electronic device 100 may support one or more video codecs. Therefore, the electronic device 100 can play or record videos of a plurality of coding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a transfer mode between human brain neurons, and may further continuously perform self-learning. The NPU can implement applications such as intelligent cognition of the electronic device, for example, image recognition, facial recognition, voice recognition, and text understanding.

The external memory interface 120 may be configured to connect to an external storage card, for example, a micro SD card, to extend a storage capability of the electronic device. The external memory card communicates with the processor 110 through the external memory interface 120, to implement a data storage function. For example, data files such as music, photos, and videos are stored in the external storage card.

The internal memory 121 may be configured to store one or more computer programs, and the one or more computer programs include instructions. The processor 110 may run the instructions stored in the internal memory 121, so that the electronic device performs a voice switching method provided in some embodiments of this application, various function applications, data processing, and the like. The internal memory 121 may include a program storage area and a data storage area. The program storage area may store an operating system. The program storage area may further store one or more applications (for example, Gallery and Contacts), and the like. The data storage area may store data (for example, Photos and Contacts) created during use of the electronic device. In addition, the internal memory 121 may include a high-speed random access memory, and may further include a nonvolatile memory, for example, at least one magnetic disk storage device, a flash memory, or a universal flash storage (universal flash storage, UFS). In some embodiments, the processor 110 may run the instructions stored in the internal memory 121 and/or instructions stored in the memory disposed in the processor 110, to enable the electronic device to perform the voice switching method provided in embodiments of this application, various function applications, and data processing.

The electronic device may implement audio functions such as music playing and recording through the audio module 170, the speaker 170A, the receiver 170B, the microphone 170C, the headset jack 170D, the application processor, and the like. The audio module 170 is configured to convert digital audio information into an analog audio signal output, and is also configured to convert an analog audio input into a digital audio signal. The audio module 170 may be further configured to encode and decode an audio signal. In some embodiments, the audio module 170 may be disposed in the processor 110, or some function modules of the audio module 170 are disposed in the processor 110.

The speaker 170A, also referred to as a "horn", is configured to convert an audio electrical signal into a sound signal. The electronic device may be used to listen to music or answer a hands-free call through the speaker 170A.

The receiver 170B, also referred to as an "earpiece", is configured to convert an audio electrical signal into a sound signal. When a call is answered or voice information is received by using the electronic device, the receiver 170B may be put close to a human ear to receive a voice.

The microphone 170C, also referred to as a "mike" or a "microphone", is configured to convert a sound signal into an electrical signal. When making a call or sending a voice message, the user may make a sound near the microphone 170C through the mouth, to enter a sound signal to the microphone 170C. At least one microphone 170C may be disposed in the electronic device. In some other embodiments, two microphones 170C may be disposed in the electronic device, to implement a noise reduction function, in addition to collecting a sound signal. In some other embodiments, three, four, or more microphones 170C may alternatively be disposed in the electronic device, to collect a sound signal and reduce noise. The microphones may further identify a sound source, to implement a directional recording function, and the like.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The sensor 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device determines strength of pressure based on a change of the capacitance. When a touch operation is performed on the display 194, the electronic device detects strength of the touch operation by using the pressure sensor 180A. The electronic device may further calculate a touch location based on a detection signal of the pressure sensor 180A. In some embodiments, touch operations that are performed at a same touch location but have different touch operation strength may correspond to different operation instructions. For example, when a touch operation whose touch operation strength is less than a first pressure threshold is performed on a Messages icon, an instruction for viewing an SMS message is executed. When a touch operation whose touch operation strength is greater than or equal to a first pressure threshold is performed on a Messages icon, an instruction for creating an SMS message is executed.

The gyro sensor 180B may be configured to determine a motion posture of the electronic device. In some embodiments, an angular velocity of the electronic device around three axes (namely, axes x, y, and z) may be determined by using the gyro sensor 180B. The gyro sensor 180B may be configured to perform image stabilization during photographing. For example, when a shutter is pressed, the gyro sensor 180B detects a jitter angle of the electronic device, calculates, based on the angle, a distance for which a lens module needs to compensate, and enables the lens to offset jitter of the electronic device through reverse motion, to implement image stabilization. The gyro sensor 180B may be further used in a navigation scenario, a motion-sensing game scenario, and the like.

The acceleration sensor 180E may detect magnitude of accelerations in various directions (usually on three axes) of the electronic device, and may detect magnitude and a direction of gravity when the electronic device is stationary. The acceleration sensor 180E may be further configured to identify a posture of the electronic device, and is used in applications such as switching between landscape mode and portrait mode or a pedometer.

The distance sensor 180F is configured to measure a distance. The electronic device may measure the distance in an infrared or a laser manner. In some embodiments, in a photographing scenario, the electronic device may measure the distance by using the distance sensor 180F, to implement quick focusing.

The optical proximity sensor 180G may include, for example, a light-emitting diode (LED) and an optical detector such as a photodiode. The light-emitting diode may be an infrared light-emitting diode. The electronic device emits infrared light by using the light-emitting diode. The electronic device detects infrared reflected light from a nearby object by using the photodiode. When detecting sufficient reflected light, the electronic device may determine that there is an object near the electronic device. When detecting insufficient reflected light, the electronic device may determine that there is no object near the electronic device. The electronic device may detect, by using the optical proximity sensor 180G, that the user holds the electronic device close to an ear for a call, to automatically turn off a screen for power saving. The optical proximity sensor 180G may also be used in a smart cover mode or a pocket mode to automatically perform screen unlocking or locking.

The ambient light sensor 180L is configured to sense ambient light brightness. The electronic device may adaptively adjust brightness of the display 194 based on the sensed brightness of the ambient light. The ambient light sensor 180L may also be configured to automatically adjust white balance during photographing. The ambient light sensor 180L may further cooperate with the optical proximity sensor 180G to detect whether the electronic device is in a pocket, so as to avoid an accidental touch.

The fingerprint sensor 180H (also referred to as a fingerprint recognizer) is configured to collect a fingerprint. The electronic device may use a feature of the collected fingerprint to implement fingerprint-based unlocking, application lock access, fingerprint-based photographing, fingerprint-based call answering, and the like. In addition, for other records about the fingerprint sensor, refer to the international patent application PCT/CN2017/082773 entitled "NOTIFICATION PROCESSING METHOD AND ELECTRONIC DEVICE".

The touch sensor 180K is also referred to as a "touch panel". The touch sensor 180K may be disposed on the display 194, and the touch sensor 180K and the display 194 form a touchscreen, which is also referred to as a "touchscreen". The touch sensor 180K is configured to detect a touch operation performed on or near the touch sensor. The touch sensor may transfer the detected touch operation to the application processor, to determine a type of a touch event. A visual output related to the touch operation may be provided through the display 194. In some other embodiments, the touch sensor 180K may alternatively be disposed on a surface of the electronic device at a location different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a human pulse, and receive a blood pressure beating signal. In some embodiments, the bone conduction sensor 180M may alternatively be disposed in the headset, to constitute a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The temperature sensor 180J may acquire temperature data. The temperature sensor 180J may include a contact temperature sensor and a non-contact temperature sensor. The contact temperature sensor needs to be in contact with a to-be-measured object, and is a heat flux sensor, a skin temperature sensor, or the like. The non-contact temperature sensor can acquire temperature data without being in contact with the to-be-measured object. It may be understood that temperature measurement principles of the temperature sensors are different. In this embodiment of this application, one or more temperature sensors may be disposed in the electronic device.

The button 190 includes a power button, a volume button, and the like. The button 190 may be a mechanical button, or may be a touch button. The electronic device may receive a key input, and generate a key signal input related to user setting and function control of the electronic device.

The motor 191 may generate a vibration prompt. The motor 191 may be configured to produce an incoming call vibration prompt and a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, a time reminder, information receiving, an alarm clock, a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or detached from the SIM card interface 195, to implement contact with or separation from the electronic device. The electronic device may support one or more SIM card interfaces. The SIM card interface 195 can support a nano SIM card, a micro SIM card, a SIM card, and the like. A plurality of cards may be simultaneously inserted into a same SIM card interface 195. The plurality of cards may be of a same type or of different types. The SIM card interface 195 is compatible with different types of SIM cards. The SIM card interface 195 is also compatible with an external storage card. The electronic device interacts with a network by using the SIM card, to implement functions such as calling and data communication. In some embodiments, the electronic device uses an eSIM, namely, an embedded SIM card. The eSIM card may be embedded into the electronic device, and cannot be separated from the electronic device.

In this embodiment of this application, the electronic device 100 may be configured to measure a body temperature of a user. Specifically, the electronic device 100 may be a smart watch, a smart band, a smart headset, smart glasses, a mobile phone, and another wearable smart device (for example, a chest strap or an arm band). This is not limited in this application.

An example in which the electronic device 100 is a smart watch is now used to describe a process in which the electronic device 100 measures the body temperature of the user.

As shown in FIG. 3, the smart watch may be worn on a wrist of the user. In this way, body temperature measurement can be completed in a process of wearing the smart watch by the user. Compared with a measurement manner in which a temperature measurement accessory is adhered to the body of the user, a possibility of skin allergy of the user caused by the adhesive manner is avoided, and comfort is high.

For example, FIG. 4 is a schematic diagram of measuring a body temperature of a user by a smart watch. As shown in FIG. 4, when the user wears the watch, the user may perform any action without limitation. When the user needs to measure the body temperature, assuming that a forehead temperature is to be measured, the user may raise a hand, to align the smart watch with the forehead of the user for short duration, for example, 1s, and the smart watch can measure the forehead temperature of the user, and display a body temperature measurement result. The body temperature measurement result may be the measured forehead temperature, or may be a temperature obtained after the forehead temperature is processed. For example, the measured forehead temperature is 36.8°C, and the displayed body temperature of the user may be 36.9°C. A manner of obtaining the body temperature measurement result is described in detail later. In addition, the body temperature measurement result may be displayed on a display of the smart watch, or may be displayed on a mobile phone (or another electronic device) connected to the smart watch. Details are described later. In this way, the user only needs to perform a simple hand raising action, and the smart watch can automatically measure the body temperature of the user. The operation is convenient and quick.

It may be understood that, in the manner shown in FIG. 4, the user may further align the smart watch with different human body parts by performing different actions, to obtain the body temperature by measuring different human body parts. For example, the user may align the smart watch with an ear to measure a tympanic membrane temperature and display the body temperature measurement result. In another example, the user may alternatively align the smart watch with an oral cavity, to measure an oral temperature and display the body temperature measurement result. In another example, the user may further align the smart watch with an axilla, to measure an axillary temperature, and further display the body temperature measurement result. Enumeration is not provided herein. The body parts measured by the user by raising the hand are correlated with the body temperature measurement results. This is described in detail later.

For ease of understanding, the technical solution provided in this embodiment of this application is subsequently described by using an example in which the user raises a hand to measure the forehead temperature.

In an embodiment, FIG. 5a, FIG. 5b, FIG. 5c, and FIG. 5d are a schematic diagram of performing human-computer interaction between a user and a smart watch to measure a body temperature. As shown in FIG. 5a, FIG. 5b, FIG. 5c, and FIG. 5d, a temperature sensor 11 and a display 15 are disposed on a front surface of the smart watch. It may be understood that the temperature sensor 11 may be one of the temperature sensors 180J shown in FIG. 2, and the display 15 may be specifically the display 194 shown in FIG. 2.

The temperature sensor 11 is disposed on the front surface of the smart watch. When the user raises the wrist, the smart watch is aligned with the forehead of the user, and the temperature sensor 11 is also aligned with the forehead of the user. In this way, the temperature sensor 11 may measure the forehead temperature when the user raises the hand, so that a current body temperature of the user can be displayed on the display 15 of the smart watch.

In a specific implementation, the temperature sensor 11 may be a non-contact temperature sensor. For example, the temperature sensor 11 may be specifically an infrared thermopile sensor. The infrared thermopile sensor is manufactured by using a principle that energy radiated by a human body to the outside varies with the temperature. Specifically, in nature, any object above absolute zero radiates energy to the outside with a particular wavelength, but wavelengths of radiation of energy to the outside are different. For example, the body temperature is 37°C, and an infrared radiation wavelength is usually 9 µm to 10 µm. The infrared thermopile sensor converts absorbed infrared radiation into heat energy (temperature) and into electronic signals for output and display. Therefore, the infrared thermopile sensor 11 may be designed on the front surface of the smart watch. In this way, as shown in FIG. 4, the user only needs to raise the wrist and does not need to be in contact with the body of the user, so that body temperature measurement can be implemented, comfort is high, and measurement scenarios are also wider.

Alternatively, in another specific implementation, the temperature sensor 11 may alternatively be a contact sensor, and the contact temperature sensor needs to be in contact with the to-be-measured object when measuring the temperature. That is, in this way, when the user raises the wrist to measure the body temperature, the smart watch may be in contact with the forehead, so that the temperature sensor 11 is in contact with the forehead of the user. In this way, the temperature sensor 11 can measure the forehead temperature, and the display 15 of the smart watch can display the current body temperature of the user. A type of the contact temperature sensor is not particularly limited in this application, and may include but is not limited to at least one of a pressure type thermometer, a resistance thermometer, a bimetallic thermometer, and a glass liquid thermometer.

The display 15 may display various contents. For example, on an interface a shown in FIG. 5a, FIG. 5b, FIG. 5c, and FIG. 5d, the display 15 may display time information. In another example, on an interface c shown in FIG. 5a, FIG. 5b, FIG. 5c, and FIG. 5d, the display 15 displays prompt information. In a specific implementation, the user may implement operation control on the smart watch by touching the display 15. For example, the user may switch, by sliding, tapping, or touching and holding with a finger, content displayed on the display 15, or control the smart watch to implement functions such as heart rate measurement and body temperature measurement.

The body temperature measurement process shown in FIG. 4 is described by using a human-computer interaction interface shown in FIG. 5a, FIG. 5b, FIG. 5c, and FIG. 5d. On the interface a shown in FIG. 5a, FIG. 5b, FIG. 5c, and FIG. 5d, the display 15 of the smart watch may display current time, which is 09:34 (24-hour format) on the morning of August 26, and today is Monday. The user may slide up and down on the display 15 to switch content displayed on the display 15. For example, after a finger of the user slides downward on the display 15 at least once, the display 15 may present an interface b shown in FIG. 5a, FIG. 5b, FIG. 5c, and FIG. 5d. On the interface b, a virtual button for body temperature measurement is displayed. The user may tap the virtual button to measure the body temperature. When the user taps the virtual button, the display 15 of the smart watch displays prompt information "Start measuring, please aim at the forehead" shown on the interface c. In this way, the user may raise the wrist, so that the smart watch is aligned with the forehead, and in this case, the temperature sensor 11 may measure the forehead temperature of the user. After the measurement is completed, the current body temperature of the user is displayed on the display 15 of the smart watch.

It may be understood that FIG. 5a, FIG. 5b, FIG. 5c, and FIG. 5d are merely an example. In an actual scenario, there may further be a plurality of changes. For example, in a possible scenario, the interface b shown in FIG. 5a, FIG. 5b, FIG. 5c, and FIG. 5d may not be displayed. When the user switches the display 15 to the body temperature measurement interface, the interface c may be directly displayed, and the user is prompted to raise a hand to perform body temperature measurement. In another example, in another possible scenario, on the interface c shown in FIG. 5a, FIG. 5b, FIG. 5c, and FIG. 5d, the user may further be prompted to align the smart watch with the ear. In this case, the temperature sensor 11 may measure a tympanic membrane temperature inside the ear.

In another possible embodiment of this application, after the smart watch measures the body temperature of the user and displays the body temperature of the user on the display 15 (an interface d shown in FIG. 5a, FIG. 5b, FIG. 5c, and FIG. 5d), the user may further return to the interface b shown in FIG. 5a, FIG. 5b, FIG. 5c, and FIG. 5d by tapping the display 15; or the user may further switch, in a manner of sliding up and down, the content displayed on the display 15 to another functional content. For example, the user may slide upward on the display 15, so that the display 15 displays content shown on the interface a. In another example, the user may slide downward on the display 15, so that the display 15 displays content related to heart rate detection.

In this embodiment of this application, there may be at least one temperature sensor disposed in the smart watch. In a specific implementation scenario, in addition to the temperature sensor 11, another temperature sensor may be further disposed in the smart watch.

For example, FIG. 6 is a schematic diagram of a structure of another electronic device. As shown in FIG. 6, a temperature sensor 12 is further disposed on a front surface of a smart watch in addition to a temperature sensor 11 and a display 15.

In this embodiment of this application, the temperature sensor 12 may be the same as the temperature sensor 11. For example, the temperature sensor 12 and the temperature sensor 11 may both be infrared thermopile sensors. In this case, the temperature sensor 12 and the temperature sensor 11 may both be disposed on the front surface of the smart watch, so that when the user raises the wrist to enable the smart watch to be aligned with the forehead of the user, the temperature sensor 12 and the temperature sensor 11 can both measure the forehead temperature. In this way, the user's body temperature displayed on the display 15 of the smart watch may be an average value of forehead temperatures measured by the two temperature sensors, or an average temperature value obtained after the two measured temperatures (the forehead temperatures measured by the temperature sensors) are corrected.

In another embodiment of this application, the temperature sensor 12 may be different from the temperature sensor 11. For example, the temperature sensor 11 may be an infrared thermopile sensor, and is configured to measure the forehead temperature when the smart watch is aligned with the forehead of the user, and the temperature sensor 12 may be configured to measure an ambient temperature. In this case, body temperature data displayed on the display 15 of the smart watch may be obtained after the forehead temperature measured by the temperature sensor 11 and the ambient temperature measured by the temperature sensor 12 are processed. A processing manner is described in detail later.

When the temperature sensor 12 is an ambient temperature sensor, the ambient temperature sensor may be disposed on the front surface of the smart watch, as shown in FIG. 6, or may be disposed on a side wall of the smart watch, as shown in FIG. 7. For example, in a specific scenario, if the temperature sensor 11 is a contact sensor, when the user raises the wrist to enable the front surface of the smart watch to be in contact with the forehead of the user, the forehead temperature can be measured. In this case, the ambient temperature sensor 12 may be disposed on the side wall of the smart watch shown in FIG. 7, to avoid a case in which the temperature sensor 12 is also in contact with the skin of the user when the smart watch is in contact with the forehead of the user, so that measurement precision of the temperature sensor 12 can be improved to some extent. In another example, in another possible scenario, if the temperature sensor 11 is an infrared thermopile sensor, the forehead temperature can be measured without contact with the forehead of the user. In this case, the ambient temperature sensor 12 may be disposed on a front surface or the side wall of the smart watch.

It should be noted that FIG. 7 shows only an example of a location at which the temperature sensors (11 and 12) are disposed in the smart watch. In an actual scenario, when the temperature sensor 11 is a non-contact sensor, an outer surface of the temperature sensor 11 may be on a same surface as an outer surface of a front housing of the smart watch, or may be disposed convexly relative to the front housing of the watch, or may be disposed concavely relative to the front housing of the watch. When the temperature sensor 11 is a contact sensor, the outer surface of the temperature sensor 11 may be on a same surface as the outer surface of the front housing of the smart watch, or may be disposed convexly relative to the front housing of the watch. When the temperature sensor 12 is an ambient temperature sensor, an outer surface of the temperature sensor 12 may be on a same surface as the outer surface of the front housing of the smart watch, or may be disposed convexly relative to the front housing of the watch, or may be disposed concavely relative to the front housing of the watch. This is not limited in this embodiment of this application. In addition, a temperature measurement principle, a model, and the like of the ambient temperature sensor 12 are not particularly limited in this application, and only a sensor that can measure an ambient temperature is needed.

In the embodiment shown in FIG. 6 or FIG. 7, the user's body temperature displayed on the display 15 may be obtained by combining the forehead temperature measured by the temperature sensor 11 and the ambient temperature measured by the temperature sensor 12. A specific processing manner is described in detail later. In this implementation, the ambient temperature is considered, so that the user's body temperature displayed on the display 15 is closer to a real core body temperature, and accuracy of a measurement result is higher.

Structures of the smart watches shown in FIG. 5a, FIG. 5b, FIG. 5c, and FIG. 5d to FIG. 7 are examples. In this embodiment of this application, actual setting locations of the temperature sensors on the front surface and the side surface of the smart watch, sizes of the temperature sensors, and data of the temperature sensors are not particularly limited. In an actual scenario, adjustment and design may be performed based on an actual product requirement.

For example, as shown in FIG. 5a, FIG. 5b, FIG. 5c, and FIG. 5d, the temperature sensor 11 may be disposed above the display 15, or may be disposed below the display 15, as shown in FIG. 7. In addition, the temperature sensor 11 may alternatively be disposed at any location on the front surface of the smart watch. In another example, in the smart watch shown in FIG. 6, the temperature sensor 11 and the temperature sensor 12 may be separately disposed above or below the display 15, or the temperature sensor 11 and the temperature sensor 12 may be disposed adjacent to each other. Enumeration is not provided herein. In addition, in some special implementation scenarios, the temperature sensor 11 and/or the temperature sensor 12 may alternatively be disposed on an outer surface of a watch strap of the smart watch, and the temperature sensor 11 and the temperature sensor 12 may be disposed adjacently or separately. The temperature sensor 11 and the temperature sensor 12 may be separately connected to a processor of the smart watch, so that the processor obtains the body temperature of the user based on measured temperature data, and displays the body temperature on the display 15. A connection manner may be wired connection, and a connection line may be buried inside the watch strap or exposed on a surface (at least one of an inner surface, an outer surface, and a side wall) of the watch strap by using other designs.

In this embodiment of this application, the body temperature measurement result may be displayed on the display 15 of the smart watch, as shown in FIG. 5a, FIG. 5b, FIG. 5c, and FIG. 5d. In addition, the body temperature measurement result may also be displayed on other electronic devices connected to the smart watch. The connection herein may include but is not limited to a wired connection or a wireless connection. For example, the smart watch may be connected to a computer by using a data line, and the body temperature measurement result of the user is displayed on the computer. In another example, the smart watch may alternatively be connected to the mobile phone by using Bluetooth, and display the body temperature measurement result of the user on a display interface of the mobile phone.

For example, FIG. 8 is a schematic diagram of displaying a body temperature measurement result of a smart watch on a mobile phone. In this case, the smart watch may be connected to the mobile phone 200 by using Bluetooth, Wi-Fi, or another manner. The smart watch may transmit the measured body temperature of the user to the mobile phone 200. In this way, the user opens a specified application (Application, APP), for example, a Health APP, and can view the body temperature of the user on a display interface of the APP.

As shown in FIG. 8, compared with the smart watch, when displaying the body temperature data of the user, the mobile phone 200 may display more body temperature data. For example, an average body temperature, a highest body temperature, and a lowest body temperature of the user in the most recent week and (a body temperature measured) at the most recent time may be displayed on the display interface shown in FIG. 8. For example, it is assumed that the user measures the body temperature of the user by raising a hand in the most recent week in the foregoing manner. The user performs measurement by raising the hand for a total of eight times, and body temperatures of the user that are obtained based on measurement data are 38°C, 37.3°C, 36.8°C, 36.5°C, 36.1°C, 36°C, 36.3°C, and 36.8°C in sequence. In this case, as shown in FIG. 8, a body temperature card may provide the user with the following information: The average body temperature of the user in the most recent week is 36.7°C, the highest body temperature of the user in the most recent week is 38°C, the lowest body temperature of the user in the most recent week is 36°C, and the body temperature measured by the user at the most recent time is 36.8°C.

Descriptions about the interface shown in FIG. 8 and touch operations are described in detail later.

This application further provides another body temperature measurement method. As shown in FIG. 9, in this method, the user does not need to raise a wrist, and in a process in which the user wears the smart watch daily, the smart watch can automatically measure the body temperature of the user. In this scenario, the user does not need to perform a specified action or operation, and the user can normally perform daily life. For the user, this body temperature measurement manner is more comfortable and convenient

During specific implementation, this solution may be implemented by using a temperature sensor disposed on a back surface (an outer surface of a side that is in contact with the skin of the wrist of the user) of the smart watch.

In a possible embodiment, refer to FIG. 10. A temperature sensor 13 is disposed on the back surface (the outer surface of the side that is in contact with the skin of the wrist of the user) of the smart watch. In this way, when the user normally wears the smart watch, the temperature sensor 13 may be in contact with the skin of the user, and may be specifically configured to measure the skin surface temperature (namely, the wrist temperature shown in FIG. 1) of the user, and the skin surface temperature may be denoted as Ts.

For example, FIG. 11 is a schematic diagram of a body temperature measurement principle according to an embodiment of this application. As shown in FIG. 11, when the user wears the smart watch, the temperature sensor 13 is in contact with the skin of the user, and the skin surface temperature Ts on the wrist of the user can be measured. In this way, the body temperature T of the user that is displayed in the smart watch or the mobile phone may be obtained after Ts is corrected.

As shown in FIG. 11, the temperature sensor 13 can implement automatic measurement. After the user triggers an automatic body temperature measurement function of the smart watch (detailed later), the smart watch can automatically measure the body temperature of the user. For example, the temperature sensor 13 may periodically measure temperature data, for example, once every other hour. In another example, the temperature sensor 13 may measure temperature data based on a preset moment. For example, the temperature sensor 13 may automatically measure temperature data at 6:00, 12:00, 16:00, 19:00, and 21:00 every day. Therefore, each piece of measured temperature data is corrected, and the body temperature data of the user at these moments can be displayed in the smart watch. For example, if the temperature sensor 13 obtains five pieces of measurement data, Ts1 to Ts5, five pieces of body temperature data, T1 to T5 can be obtained after correction processing. In this way, the temperature T obtained after Ts is corrected may be displayed on the display 15 of the smart watch or the mobile phone 200 connected to the smart watch.

In addition, there may be one or more temperature sensors 13, and a location of the third sensor 13 may be any location on the back surface of the smart watch or any location on the back surface of the watch strap.

In the embodiment shown in FIG. 10, the smart watch may implement continuous measurement of the body temperature of the user, so that a requirement of continuous measurement of the body temperature in scenarios such as female physiological cycle management, biological rhythm adjustment, and chronic disease management can be met. Moreover, this measurement process is convenient and comfortable for the user. In addition, in this embodiment of this application, measurement precision of the body temperature measurement result output by the smart watch is further improved to some extent by correcting the measured body temperature.

In addition, FIG. 11 further shows a relationship between the skin surface temperature (denoted as Ts) and the deep tissue temperature (denoted as Td) of the human body. For a human body, the skin surface temperature Ts is usually different from the deep tissue temperature Td, and usually, the deep tissue temperature Td is higher than the skin surface temperature Ts. A temperature difference between the skin surface temperature Ts and the deep tissue temperature Td is related to a heat flux HF. The heat flux may also be referred to as thermal flux or heat flux density, which refers to heat energy passing through a particular area per unit time, and is a vector with directivity. A unit of the heat flux in the international system of units is Joule per second (J/s), that is, Watt (W). As shown in FIG. 12, the skin surface temperature Ts, the deep tissue temperature Td, and the heat flux HF may satisfy the following relationship: Td=Ts+HF*R, where R represents skin thermal resistance. Thermal resistance is a ratio of a temperature difference between two ends of an object to a power of a heat source when heat is transmitted over the object, and has a unit of kelvin per watt (K/W) or degree Celsius per watt (°C/W). For example, the skin thermal resistance of the human body may be. In this way, when the skin surface temperature Ts and the heat flux HF are measured, the deep tissue temperature Td can be obtained; or, when the deep tissue temperature Td and the heat flux HF are measured, the skin surface temperature Ts can be obtained.

Therefore, in this embodiment of this application, the deep tissue temperature Td may alternatively be measured and corrected, to display a temperature obtained after Td is corrected.

FIG. 12 and FIG. 13 show this design. A temperature sensor 14 is disposed on the back surface (the outer surface of the side that is in contact with the skin of the wrist of the user) of the smart watch. In this way, when the user normally wears the smart watch, the temperature sensor 14 may be in contact with the skin of the user, and may be specifically configured to measure the deep tissue temperature Td or the heat flux HF of the user.

In the embodiment shown in FIG. 12, the temperature sensor 14 may be configured to measure the deep tissue temperature Td. In a possible embodiment, two temperature measurement modules and a processor may be disposed in the temperature sensor 14. One measurement module may be configured to measure the skin surface temperature Ts, and the other measurement module is configured to measure the heat flux HF. The processor may be configured to: obtain the deep tissue temperature Td on the wrist of the user based on Ts and HF, and output Td. When the user wears the smart watch, the temperature sensor 14 is in contact with the skin of the user, and can measure the deep tissue temperature Td on the wrist of the user. In this way, the smart watch may display the temperature T obtained after Td is corrected.

In this case, the temperature sensor 14 may be a heat flux sensor. The heat flux sensor, also referred to as a thermal flux sensor, may be configured to measure a deep temperature of an object. A measurement principle of the heat flux sensor is that the deep temperature of the object can be obtained by measuring a heat flux between the depth and the surface of the object in combination with the surface temperature of the object. Therefore, only one heat flux sensor may be disposed on the back surface of the smart watch, so that the deep tissue temperature Td of the user can be measured.

In the embodiment shown in FIG. 13, the temperature sensor 14 may also be a heat flux sensor, but the heat flux sensor may be configured to measure the heat flux HF, or configured to measure the deep tissue temperature Td.

The temperature sensor 14 may be configured to measure the heat flux HF. In this case, the skin surface temperature Ts measured by the temperature sensor 13 is also needed to obtain the deep tissue temperature Td. Therefore, in this embodiment, the temperature sensor 13 and the temperature sensor 14 are disposed on the back surface of the smart watch. After obtaining Ts and HF, the processor of the smart watch may determine Td, and further display the body temperature T obtained after Td is corrected.

Alternatively, the temperature sensor 14 may be configured to measure the deep tissue temperature Td. In this case, the smart watch may determine the body temperature T of the user based on the skin surface temperature Ts and the deep tissue temperature Td. For example, the smart watch may perform preliminary correction on the deep tissue temperature Td by using the skin surface temperature Ts and preset HF, to obtain Td', so that the body temperature T obtained after Td' is corrected is displayed in the smart watch. When Td is preliminarily corrected by using Ts and HF, a deep tissue temperature Td" corresponding to Ts and HF may be obtained based on the manner shown in FIG. 11, to obtain an average value (or a weighted average value) of Td" and Td, to obtain corrected Td'.

Compared with the skin surface temperature Ts, the deep tissue temperature Td changes less and is relatively more stable. Therefore, a correspondence between the deep tissue temperature Td and the body temperature T of the user is also more stable. Therefore, in the embodiments shown in FIG. 12 and FIG. 13, when the body temperature T is obtained by using Td, accuracy of a correction result can be improved to some extent, and a degree of closeness between the body temperature T and an actual body temperature of the user is improved. This helps to obtain a body temperature measurement result with higher accuracy, and also has higher stability.

In addition, in the embodiments shown in FIG. 12 and FIG. 13, continuous measurement, for example, periodic measurement or timing measurement, can also be implemented, to meet a requirement of continuous measurement of the body temperature in scenarios such as female physiological cycle management, biological rhythm regulation, and chronic disease management. Similarly, this measurement process is also convenient and comfortable for the user.

It should be noted that, in this embodiment of this application, a temperature sensor may be separately disposed on the front surface of the smart watch. For example, in the embodiments shown in FIG. 4 to FIG. 8, the user may actively raise a hand to measure the body temperature of the user. Alternatively, a temperature sensor may be separately disposed on the back surface of the smart watch. For example, in the embodiments shown in FIG. 9 to FIG. 13, after the user enables the automatic body temperature measurement function, the smart device 100 can automatically measure the body temperature of the user.

In an actual implementation scenario, the foregoing two measurement manners may be combined.

For example, FIG. 14 is a schematic diagram of a structure of another smart watch. As shown in FIG. 14, four temperature sensors are disposed in the smart watch: a temperature sensor 11 disposed on a front surface of the smart watch and configured to measure a temperature of a forehead (inside an ear, or the like) of the user; a temperature sensor 12 disposed on a side surface of the smart watch and configured to measure an ambient temperature; a temperature sensor 13 disposed on a back surface of the smart watch and configured to measure a skin surface temperature of the user; and a temperature sensor 14 disposed on the back surface of the smart watch and configured to measure a deep tissue temperature of the user. In addition, the display 15 is further disposed on the front surface of the smart watch.

It may be understood that FIG. 14 also shows locations of the temperature sensors only by using an example. In an actual scenario, the temperature sensor 13 may be configured to measure the skin surface temperature of the user, and the temperature sensor 14 may be configured to measure the deep tissue temperature or a heat flux of the user. Therefore, outer surfaces of the two temperature sensors may be on a same surface as an outer surface of a back housing of the smart watch, or may be disposed convexly relative to the back housing of the watch.

In the smart watch shown in FIG. 14, each temperature sensor may measure temperature data in the manner described in the foregoing embodiments, and the smart watch may also display a body temperature measurement result (body temperature T) of the user in the manner described in the foregoing embodiments. Details are not described.

In addition, in this embodiment, the body temperature T of the user that is displayed in the electronic device 100 may alternatively be obtained through calculation based on measurement data of each temperature sensor.

For example, refer to FIG. 15. FIG. 15 is a schematic diagram of another body temperature measurement manner according to the embodiment shown in FIG. 14. In one aspect, the temperature sensor 11 may measure the forehead temperature Tf of the user when the user raises the wrist, and the temperature sensor 12 may measure the ambient temperature Te. Then, the forehead temperature Tf may be corrected by using the ambient temperature Te, to obtain a forehead body temperature Tc of the user. In a further aspect, the temperature sensor 13 may measure the skin surface temperature Ts of the user, and the temperature sensor 14 may be configured to measure a heat flux HF between the skin surface and the deep tissue. In this way, the deep tissue temperature Td of the user can be obtained. In this way, the forehead body temperature Tc may be used to correct the deep tissue temperature Td, to obtain a body temperature measurement result of the user and display the result. A specific correction manner is described in detail later.

In the smart watch shown in FIG. 14, temperature measurement results of a plurality of temperature sensors may be combined to finally determine the body temperature T of the user, so that accuracy of the determined body temperature T can be improved to some extent.

In this embodiment of this application, in the embodiments shown in FIG. 10 to FIG. 15, the body temperature measurement result obtained by the smart watch may be displayed on the display 15 of the smart watch, or may be displayed on the mobile phone 200 connected to the smart watch. Specifically, the body temperature measurement result may be displayed on a display interface of the Health APP of the mobile phone 200.

For example, FIG. 16a, FIG. 16b, FIG. 16c, and FIG. 16d are a schematic diagram of a human-computer interaction scenario.

When the user opens the Health APP, the mobile phone may present an interface a shown in FIG. 16a, FIG. 16b, FIG. 16c, and FIG. 16d. The interface a includes a plurality of tab page controls (for example, a home page control 201), a tab bar 202, a body temperature card 203, a heart rate card 204, and a card management control 205. The home page control 201 is in a selected state, to prompt the user that the user is currently on an APP home page interface. In addition, if the user taps another control, the user may access a display interface corresponding to a tab page control. For example, if the user taps a device control, corresponding content of a device tab page is displayed on a display interface of the mobile phone 200. A display manner of content corresponding to each tab page and the content are not limited in this embodiment of this application.

On the interface a in FIG. 16a, FIG. 16b, FIG. 16c, and FIG. 16d, the tab bar 202 may be used to display device information. For example, the content that may be displayed includes but is not limited to: operator information (China Mobile), operator signal strength, current time information (09:34), and battery level information. In addition, the tab bar 202 may further display user information, for example, at least one of a user headshot and a user name. The user headshot and the user name displayed herein may be default settings of the Health APP, or may be customized or set by the user. In addition, a connection status control 2021 is further set in the tab bar 202, and the connection status control 2021 is used to indicate a connection status between the mobile phone 200 and the smart watch. For example, when the mobile phone 200 is connected to the smart watch by using Bluetooth, the connection status control 2021 displays "connected". When the Bluetooth connection between the mobile phone 200 and the smart watch is disconnected, the connection status control 2021 may display "not connected". It may be understood that the tab bar 202 may display more or less content. For example, a Wi-Fi connection status may be further displayed. In another example, current time information may not be displayed in the tab bar 202.

The body temperature card 203 is used to display the body temperature of the user. As shown in the interface a in FIG. 16a, FIG. 16b, FIG. 16c, and FIG. 16d, a plurality of pieces of body temperature data: an average body temperature, (a body temperature measured) at the most recent time, a highest body temperature, and a lowest body temperature may be displayed on the body temperature card 203. The average body temperature, the highest body temperature, and the lowest body temperature may be data statistical results in a same time interval. For example, on the interface a in FIG. 16a, FIG. 16b, FIG. 16c, and FIG. 16d, the body temperature card 203 may display body temperature statistical data of the user in the most recent day. There may also be another manner, which is described in detail later.

The heart rate card 204 is used to display heart rate data of the user. As described on the interface a in FIG. 16a, FIG. 16b, FIG. 16c, and FIG. 16d, the heart rate card may display a plurality of pieces of heart rate data: a resting heart rate, (a heart rate measured) at the most recent time, a highest heart rate, and a lowest heart rate.

The card management control 205 is used to manage each content card displayed on the home page. By using the card management control 205, the user may adjust which content cards are displayed on the home page interface. For example, by using the card management control 205, the user may add other content cards, such as a sleep card (used to display a sleep status of the user) and a sports card (used to display information such as a quantity of movement steps, running duration, and a running distance of the user) on the home page interface, and the user may further delete the heart rate card 204 from the home page interface. In this way, the heart rate status of the user is no longer displayed on the home page interface. In addition, by using the card management control 205, the user may further adjust a display order of the content cards. For example, the heart rate card 204 may be displayed above the body temperature card 203.

The user may tap the body temperature card 203 on the interface a, so that the mobile phone 200 displays an interface b shown in FIG. 16a, FIG. 16b, FIG. 16c, and FIG. 16d, and the user may view details of the body temperature of the user on the interface b.

A time interval 206, a body temperature curve 207, and statistical information 208 may be displayed on the interface b shown in FIG. 16a, FIG. 16b, FIG. 16c, and FIG. 16d.

For example, the time interval 206 may display one or more time intervals. For example, a body temperature condition of a specific time interval, for example, the most recent week, may be displayed by default. In another example, the interface b shown in FIG. 16a, FIG. 16b, FIG. 16c, and FIG. 16d may display a plurality of different time intervals. For example, a time interval indicated by "day" is a most recent day. Specifically, the time interval may be the most recent 24 hours, or may be a natural day, and is still a time interval from 0:00 to 23:59 each day. In addition, the time interval in FIG. 16a, FIG. 16b, FIG. 16c, and FIG. 16d is an example, and should not be construed as a limitation in an actual scenario. For example, the time interval may further be set to the most recent three days. This all falls within the scope of this application.

A vertical coordinate of the body temperature curve 207 is a body temperature, and a horizontal coordinate is time, and the body temperature curve 207 is used to indicate a change status of the body temperature of the user in a current time interval. The statistical information 208 displays statistical data of the body temperature of the user in the current time interval, and specifically displayed content may include but is not limited to: an average body temperature, a highest body temperature, a lowest body temperature, and a most recent body temperature. For example, on the interface b shown in FIG. 16a, FIG. 16b, FIG. 16c, and FIG. 16d, the currently selected time interval is "day", and the body temperature curve 207 displays a body temperature change curve of the user in the most recent day. Body temperature information displays information about the average body temperature, the highest body temperature, the lowest body temperature, the most recent body temperature, and whether abnormality occurs of the user in this day.

In this embodiment of this application, the statistical information 208 further displays abnormality prompt information 2081. When the body temperature of the user is abnormal, "whether an abnormality exists: Yes" is displayed. Otherwise, if there is no abnormality, "whether an abnormality exists: No" is displayed. During specific implementation, the body temperature of the user in the current time interval may be compared with a preset body temperature threshold, and whether the body temperature of the user is abnormal is determined. For example, a high temperature threshold, for example, 37.3°C may be preset. Therefore, as shown on the interface b, the highest body temperature of the user in the most recent day is 38°C, which is greater than the high temperature threshold, and it is determined that the body temperature of the user is abnormal. In an actual scenario, the body temperature threshold may be set to at least one of the high temperature threshold and a low temperature threshold. It may be understood that if the body temperature of the user is less than (or equal to) the low temperature threshold, it may be determined that the body temperature of the user is abnormal. It should be noted that the abnormality prompt information 2081 may be implemented after a body temperature abnormality reminding function is enabled. If the function is not enabled, the abnormality prompt information 2081 may not be displayed, or, for example, the abnormality prompt information 2081 may be displayed as "the abnormality prompt function is not enabled; you can enable this function on a device setting page". A method for enabling the body temperature abnormality reminding function is described in detail later (FIG. 19a, FIG. 19b, FIG. 19c, and FIG. 19d).

In this embodiment of this application, the user may adjust the time interval 206, so that the body temperature curve 207 and the statistical information 208 display body temperature data in different time intervals. The user may perform a touch operation through tapping (or sliding, speech instructions, gesture actions, and the like), to adjust the time interval. For example, the user may tap "week", and the mobile phone 200 may display an interface c shown in FIG. 16a, FIG. 16b, FIG. 16c, and FIG. 16d. The body temperature change curve and statistical data of the user in the most recent week are displayed on the interface c. Details are not described. Similarly, the user may further perform further adjustment on the interface c, to view detailed body temperature data of the user in the most recent month or the most recent year.

After viewing the detailed body temperature data, the user may further return to the home page interface of the Health APP. For example, a return control 209 on the interface c may be tapped to return to the home page interface. In another example, the user may alternatively return to the home page interface by using a speech instruction, a specified action gesture (for example, drawing a shape "C"), a hidden return button, or the like.

As shown in FIG. 16a, FIG. 16b, FIG. 16c, and FIG. 16d, after tapping the return control 209, the user may enter an interface d shown in FIG. 16a, FIG. 16b, FIG. 16c, and FIG. 16d. In this case, a time interval corresponding to body temperature data displayed on the body temperature card 203 displayed on the home page interface is consistent with a time interval on a body temperature details interface. Therefore, the body temperature data displayed on the body temperature card 203 is the same as the statistical information 208 displayed on the body temperature details interface. For example, in FIG. 16a, FIG. 16b, FIG. 16c, and FIG. 16d, data displayed on the body temperature card 203 on the interface a is consistent with data displayed by the statistical information 208 on the interface b. In another example, in FIG. 16a, FIG. 16b, FIG. 16c, and FIG. 16d, statistical information on the interface c is also consistent with data displayed on the body temperature card on the interface d.

Therefore, in the implementation scenario shown in FIG. 16a, FIG. 16b, FIG. 16c, and FIG. 16d, the time interval of the body temperature card 203 displayed on the home page interface may be adjusted by adjusting the time interval 206 on the body temperature details interface.

In addition, in this embodiment of this application, the user may alternatively directly adjust the time interval of the body temperature card on the home page interface. Refer to a schematic diagram of another human-computer interaction scenario shown in FIG. 17a, FIG. 17b, FIG. 17c, and FIG. 17d.

An interface a shown in FIG. 17a, FIG. 17b, FIG. 17c, and FIG. 17d is a home page interface of the Health APP, and a body temperature card 203 is displayed on the home page interface. In this embodiment of this application, a time control 2031 is displayed on the body temperature card 203, and the time control 2031 may be used to display a time interval corresponding to body temperature data currently displayed on the body temperature card 203. For example, the body temperature card 203 on the interface a displays body temperature statistical data of the most recent week.

The user may perform a touch operation on the time control 2031, to adjust the time interval. As shown in FIG. 17a, FIG. 17b, FIG. 17c, and FIG. 17d, the user may tap the time control 2031 on the interface a. In this case, a selection bar 2032 that displays the time interval, a cancellation control 2033, and an OK control 2034 may be superimposed on the current interface, as shown on an interface b in FIG. 17a, FIG. 17b, FIG. 17c, and FIG. 17d. As shown in FIG. 17a, FIG. 17b, FIG. 17c, and FIG. 17d, a plurality of different time intervals are displayed on the selection bar 2032, and the user may perform sliding selection on the selection bar, to determine a time interval that the user desires. For example, the user slides in the selection bar 2032, and adjusts the currently selected time interval "the most recent week" to "the most recent day". Therefore, on an interface c, if the user taps the cancellation control 2033, the mobile phone 200 further displays the interface a, and still displays body temperature statistical data of the user in the most recent week. Alternatively, if the user further taps the OK control 2034, as shown in FIG. 17a, FIG. 17b, FIG. 17c, and FIG. 17d, an interface d is displayed in the mobile phone 200, and a body temperature card on the interface d displays body temperature statistical data of the user in the most recent day. In this way, the user can directly adjust the time interval of the body temperature card 203 on the home page interface.

In this embodiment of this application, the embodiment shown in FIG. 17a, FIG. 17b, FIG. 17c, and FIG. 17d and the embodiment shown in FIG. 16a, FIG. 16b, FIG. 16c, and FIG. 16d may be implemented independently. Alternatively, the embodiment shown in FIG. 17a, FIG. 17b, FIG. 17c, and FIG. 17d and the embodiment shown in FIG. 16a, FIG. 16b, FIG. 16c, and FIG. 16d may be combined.

For example, in an embodiment, the time control 2031 displayed in the body temperature card 203 is only used to prompt the user. In this case, the user cannot directly adjust the time interval of the body temperature card on the home page interface by using the method shown in FIG. 17a, FIG. 17b, FIG. 17c, and FIG. 17d. Alternatively, the time control 2031 may not be displayed in the body temperature card 203. The user may adjust, in the manner shown in FIG. 16a, FIG. 16b, FIG. 16c, and FIG. 16d, a time interval of data displayed by the body temperature card 203.

In another example, in another embodiment, the time interval corresponding to the body temperature card may not be synchronized with the time interval on the body temperature details interface. In this case, the user may adjust the time interval corresponding to the body temperature card on the home page interface in the manner shown in FIG. 17a, FIG. 17b, FIG. 17c, and FIG. 17d. In addition, the user may arbitrarily switch the time interval 206 on the body temperature details interface, for example, the interface b shown in FIG. 16a, FIG. 16b, FIG. 16c, and FIG. 16d, but the switching does not act on the time interval of the body temperature card. For example, the time interval of the home page card may be the most recent week, and the time interval viewed by the user on the body temperature details interface may be "year" (that is, the most recent year).

In another example, in another embodiment, the manners shown in FIG. 16a, FIG. 16b, FIG. 16c, and FIG. 16d and FIG. 17a, FIG. 17b, FIG. 17c, and FIG. 17d may be combined. In this case, the user may tap the time control 2031 on the home page interface to adjust the time interval, and the user may alternatively adjust the time interval 206 on the body temperature details interface. These adjustments are synchronized on the body temperature card and the body temperature details page.

In another embodiment, the time interval corresponding to the body temperature card 203 may alternatively be a default design, and cannot be adjusted by the user. For example, the body temperature card 203 may display body temperature data of the most recent day by default. The scenario shown in FIG. 16a, FIG. 16b, FIG. 16c, and FIG. 16d is used as an example. In this case, if the user taps the return control 209 on the interface c, the user returns to the interface a shown in FIG. 16a, FIG. 16b, FIG. 16c, and FIG. 16d.

In addition, in this embodiment of this application, the electronic device 100 may alternatively not provide the user with a permission or function of adjusting the time interval. In this case, the time interval displayed by the body temperature card may be determined by the electronic device based on system presettings or use data of the user.

In an embodiment, when the user opens the Health APP, for example, on the interface a in FIG. 16a, FIG. 16b, FIG. 16c, and FIG. 16d, the time interval corresponding to the body temperature card may be a preset time interval. For example, when the Health APP is started, the body temperature card displays temperature data of the most recent day by default.

In another embodiment, when the user opens the Health APP, the time interval corresponding to the body temperature card may be a corresponding time interval when the user closes the Health APP last time. For example, if the user finally views body temperature data of the user in the most recent week when the user opens the Health APP for the first time to view body temperature detailed information of the user, in this case, when the user opens the Health APP for the second time, the body temperature data of the most recent week may be displayed on the interface a.

In another embodiment, when the user opens the Health APP, on the interface a in FIG. 16a, FIG. 16b, FIG. 16c, and FIG. 16d, the time interval corresponding to the body temperature card may be a time interval that the user views for a largest quantity of times. For example, the user uses the Health APP to view the body temperature data of the user for a total of 10 times in the most recent month (switching is not considered). The user views daily body temperature changes of the user for five times, views weekly body temperature changes for four times, and views monthly body temperature changes for one time. Then, when the user opens the Health APP again, the body temperature data of the user in the most recent day is displayed on the interface a.

In addition, the heart rate card 204 may also be designed with reference to any one of the foregoing designs of the body temperature card 203, and details are not described.

In this embodiment of this application, the body temperature display manners shown in FIG. 16a, FIG. 16b, FIG. 16c, and FIG. 16d and FIG. 17a, FIG. 17b, FIG. 17c, and FIG. 17d may be applicable to any one of the foregoing embodiments in FIG. 3 to FIG. 15.

In the body temperature measurement method in any embodiment of FIG. 9 to FIG. 15, the smart watch may automatically measure the body temperature of the user. In a specific implementation scenario, the automatic body temperature measurement function may be enabled by default. That is, the smart watch can automatically measure the body temperature of the user based on a preset period or a preset moment without performing an additional operation by the user.

In another embodiment, the user may decide whether to enable the automatic body temperature measurement function. In this case, reference may be made to another human-computer interaction scenario shown in FIG. 18a, FIG. 18b, FIG. 18c, and FIG. 18d.

After opening the Health APP of the mobile phone 200, the user may enter a home page interface. In this case, the user may tap a device control 210, so that the mobile phone displays an interface a shown in FIG. 18a, FIG. 18b, FIG. 18c, and FIG. 18d. The interface is a tab page interface on which the device control 210 is in a selected state.

On the interface a of FIG. 18a, FIG. 18b, FIG. 18c, and FIG. 18d, in addition to a plurality of tab page controls, a device card 211 and a tab bar 212 are further displayed. There may be one or more device cards 211. FIG. 18a, FIG. 18b, FIG. 18c, and FIG. 18d shows device cards of three smart wearable devices. Specifically, the device card 211 may include a device name 2111, a device icon 2112, a Bluetooth connection status 2113, and a device battery level 2114. The device name 2111 and the device icon 2112 may be default settings, or may be customized and edited by the user. The Bluetooth connection status 2113 is used to represent a Bluetooth connection status between the smart watch and the mobile phone 200. For example, a smart watch "Honor Watch Magic" is currently connected to the mobile phone, and a Bluetooth connection between a smart watch "Huawei Watch GT" and the mobile phone is disconnected, and the smart watch "Huawei Watch GT" is in a disconnected state. The device battery level 2114 is a battery level of the smart watch, and is not a battery level of the mobile phone 200. The battery level of the mobile phone 200 is displayed in the tab bar 211. For example, a battery level of a smart band "Honor Band 3-4fd" is 80%, and a battery level of "Honor Watch Magic" is 50%. When the smart wearable device is not connected to the mobile phone, the device battery level may not be displayed, as shown by the device card of "Huawei Watch GT".

The user may tap a device card of any smart wearable device, to set the smart wearable device. As shown in FIG. 18a, FIG. 18b, FIG. 18c, and FIG. 18d, the user taps the smart card 211 of "Honor Watch Magic", to enter a device setting interface of the smart watch, that is, an interface b in FIG. 18a, FIG. 18b, FIG. 18c, and FIG. 18d. On the interface b in FIG. 18a, FIG. 18b, FIG. 18c, and FIG. 18d, the user may enable or disable a function of the smart watch. For example, the user may enable a sedentary reminding function of the smart watch. In another example, the user may disable an automatic heart rate measurement function. The user may enable or disable the automatic body temperature measurement function. On the interface b, the automatic body temperature measurement function is currently in a "disabled" state, and the user may tap a body temperature setting control 213, so that the mobile phone can jump to a body temperature setting interface, that is, an interface c. On the interface c, the automatic body temperature measurement function provided by the smart watch is described. For example, the interface c specifically displays a wearing manner of the smart watch and text description: "Intelligently monitor your body temperature for 24 hours. For accurate measurement, please wear your watch tightly." For example, the interface c further displays reminding information for the user, and details are not described. Through the related description on the interface c, the user may learn about the automatic body temperature measurement function and determine, based on a requirement of the user, whether to enable the function. When the user wants to enable the automatic body temperature measurement function, the user only needs to tap an enable control 214 on the interface c. In this case, on a setting interface (an interface d) of the smart watch, the automatic body temperature measurement function is in an "enabled" state. In this way, the smart watch can automatically measure the body temperature of the user.

It may be understood that, if the automatic body temperature measurement function is in the "enabled" state on the setting interface of the smart watch, the user may also tap the body temperature setting control 213 to enter the body temperature setting interface, and further tap a disable control (not shown in FIG. 18a, FIG. 18b, FIG. 18c, and FIG. 18d) to disable the function.

In addition, the user may further tap a body temperature abnormality reminding control 215 on a device setting interface, for example, an interface a shown in FIG. 19a, FIG. 19b, FIG. 19c, and FIG. 19d, to enter an interface b shown in FIG. 19a, FIG. 19b, FIG. 19c, and FIG. 19d, and enable or disable a body temperature abnormality reminding function on the interface b. As shown in FIG. 19a, FIG. 19b, FIG. 19c, and FIG. 19d, the interface b displays: a control 216 for controlling enabling/disabling of a body temperature abnormality reminding function, a control 217 for controlling enabling/disabling of a default reminding function, and a control 218 for controlling enabling/disabling of a function of manually setting a warn threshold. It may be understood that an enabling or disabling operation can be performed on the control 217 and the control 218 only when the control 216 is in an enabled state. In addition, only one of the control 217 and the control 218 can be enabled. That is, body temperature abnormality reminding can be performed only based on a default threshold (when the control 217 is enabled), or reminding is performed based on a warn threshold set by the user (when the control 218 is enabled).

On the interface b shown in FIG. 19a, FIG. 19b, FIG. 19c, and FIG. 19d, the body temperature abnormality reminding function is currently in a disabled state, and the user may tap the control 216. In this case, refer to an interface c, and the body temperature abnormality reminding function is enabled. In this case, the user may tap the control 217 or the control 218. If the user taps the control 218, the user may perform a touch operation on a control 2181, to set a body temperature abnormality reminding upper limit that the user desires, and/or, the user may also perform a touch operation on a control 2182, to set a body temperature abnormality reminding lower limit that the user desires. For example, if the user sets the body temperature abnormality reminding upper limit to 37.3°C and sets the body temperature abnormality reminding lower limit to 36.2°C, the setting interface may be converted from the interface c to an interface d. After the user completes setting, the smart watch or the mobile phone may perform body temperature abnormality reminding based on the preset body temperature abnormality reminding upper limit threshold and/or lower limit threshold, as shown on the interface b in FIG. 16a, FIG. 16b, FIG. 16c, and FIG. 16d, and details are not described.

The body temperature measurement methods provided in the embodiments of this application are specifically described now.

In a possible implementation scenario, when the infrared thermopile sensor 11 is disposed on the front surface of the smart watch, for example, as shown in FIG. 5a, FIG. 5b, FIG. 5c, and FIG. 5d to FIG. 7, and FIG. 14, FIG. 20 is a schematic diagram of a body temperature measurement method in this case.

When the user raises the wrist, so that the infrared thermopile sensor 11 disposed on the front surface of the smart watch is aligned with the forehead of the user, the infrared thermopile sensor 11 can measure the temperature Tf on the forehead of the user, and transmit the measured temperature Tf to the processor 110, so that after receiving the measured temperature Tf, the processor 110 may directly output Tf.

It should be noted that, the processor 110 may output Tf to the display 15, so that Tf can be displayed on the display of the smart watch. Alternatively, when the smart watch is connected to the mobile phone 200 by using Bluetooth, Tf may also be output to the mobile phone, so that Tf can be displayed in the Health APP of the mobile phone. Details are not described later.

In another possible implementation scenario, when the infrared thermopile sensor 11 is disposed on the front surface of the smart watch, and the ambient temperature sensor 12 is disposed on the front surface or the side surface of the smart watch, for example, as shown in FIG. 6, FIG. 7, and FIG. 14, FIG. 21 is a schematic diagram of a body temperature measurement method in this scenario.

When the user raises the wrist, so that the infrared thermopile sensor 11 disposed on the front surface of the smart watch is aligned with the forehead of the user, the infrared thermopile sensor 11 can measure the forehead temperature Tf, and the ambient temperature sensor 12 can measure the current ambient temperature Te. In this way, after receiving the measured temperatures Tf and Te, the processor 110 corrects Tf by using Te to obtain the corrected temperature Tc. In this way, Tc is output and displayed.

In a possible embodiment, a correspondence among the measured temperatures Tf, Te, and Tc may be preset. For example, a temperature correspondence table may be established in advance. In this way, when receiving Tf and Te, the processor 110 may determine, by querying the table, a value of Tc corresponding to the measured temperatures, and output the value of Tc.

**Table 1**

| | | |
|---|---|---|
| Ambient temperature Te (°C) | 25 | 30 |
| Forehead temperature Tf (°C) | 36.6 | 36.6 |
| Body temperature Tc (°C) on the forehead after correction | 36.8 | 36.7 |

For example, reference may be made to the temperature correspondence table shown in Table 1. For example, in an embodiment, when the temperature (Tf) measured by the temperature sensor 11 is 36.6°C and the temperature (Te) measured by the temperature sensor is 25°C, Table 1 may be queried to learn that the corrected body temperature (Tc) on the forehead is 36.8°C, and 36.8°C is displayed on the display 15 of the smart watch.

In another example, the processor 110 may acquire sample data of Tf, Te, and Tc, perform curve fitting on the sample data, to obtain a mathematical formula among the three, and use the mathematical formula as the correspondence. In this way, when the processor receives Tf and Te, a corresponding Tc value can be obtained by substituting the values into the mathematical formula. The correspondence or the correspondence table among the three may be stored at any location that can be invoked by the processor, for example, stored in the internal memory 121.

In addition, the body temperature measurement method shown in FIG. 21 is further applicable to a possible case: When the infrared thermopile sensor 11 is disposed on the front surface of the smart watch, but the ambient temperature sensor 12 is not disposed, the processor 110 may receive Tf, and obtain the ambient temperature in another manner. Further, the processor 110 may alternatively determine and output Tc in the manner shown in FIG. 21.

In an embodiment, a default ambient temperature may be preset in the processor 110. For example, the ambient temperature may be 25°C by default for processing. In another example, the ambient temperature in summer (June to September) may be set to 30°C, and the ambient temperature in winter (December to February) may be set to 15°C. In this case, the processor 110 may select different ambient temperatures based on a current moment for processing.

In another embodiment, the smart watch may alternatively obtain, in a communication manner, a current temperature recorded on the network, as the ambient temperature. For example, the smart watch may communicate with the mobile phone 200 by using Bluetooth. During a Bluetooth connection, the mobile phone 200 may send, to the smart watch, temperature data on that day recorded by a weather module of the mobile phone 200. In this case, the temperature data sent by the mobile phone 200 may be one piece of temperature data, for example, 23°C, or may be a temperature table, which indicates weather conditions at various moments, for example, 23°C at 12:00 and 21°C at 14:00. In another example, the smart watch may access a network by using Wi-Fi, or access a cellular network by using an LTE (Long Term Evolution, long term evolution) module, to perform processing shown in FIG. 21 by using temperature data provided by a weather server as the ambient temperature. The LTE module may support an LTE network and an evolved network of the LTE network, for example, a 3G network, a 4G network, and a 5G network.

In a possible implementation scenario, one or more temperature sensors are disposed on the back surface of the smart watch. For example, the surface temperature sensor 13 is disposed on the back surface of the smart watch shown in FIG. 10. In another example, the heat flux sensor 14 is disposed on the back surface of the smart watch shown in FIG. 11. In another example, the surface temperature sensor 13 and the heat flux sensor 14 are disposed on the back surface of the smart watch shown in FIG. 13 or FIG. 14.

In this case, the processor may receive the temperature measured by the temperature sensor, and directly output the received temperature. For example, in the scenario shown in FIG. 10, the smart watch may measure the skin surface temperature Ts by using the surface temperature sensor 13, and display the skin surface temperature Ts on the display 15. In another example, in the scenario shown in FIG. 11, the smart watch may measure the deep tissue temperature Td by using the heat flux sensor 14, and display the deep tissue temperature Td on the display 15.

However, in the scenario shown in FIG. 13 or FIG. 14, after receiving the skin surface temperature Ts and the heat flux HF that are measured by the temperature sensors, the processor may further calculate the deep tissue temperature Td, and output the deep tissue temperature Td. In this way, the deep tissue temperature Td can be displayed on the display 15 of the smart watch.

In another possible implementation scenario, the infrared thermopile sensor 11 is disposed on the front surface of the smart watch, as shown in FIG. 5a, FIG. 5b, FIG. 5c, and FIG. 5d to FIG. 7, and FIG. 14. In addition, the surface temperature sensor 13 is further disposed on the back surface of the smart watch, for example, as shown in FIG. 10, FIG. 13, and FIG. 14.

In this case, FIG. 22 is a schematic diagram of a body temperature measurement method in this scenario.

Specifically, the infrared thermopile sensor 11 works when the user raises the wrist to be aligned with the forehead, and the surface temperature sensor 13 may acquire the skin surface temperature of the user periodically or in a timing manner. Because the skin surface temperature acquired by the surface temperature sensor 13 may not be stable, and may differ greatly from the body temperature of the human body, the temperature at the forehead may be used to correct Ts, to obtain a body temperature measurement result closer to the actual body temperature of the human body.

As shown in FIG. 22, the processor 110 may receive measurement data Tf and Ts acquired respectively by the infrared thermopile sensor 11 and the surface temperature sensor 13, and correct Ts by using a first correspondence, to obtain the body temperature T of the user and then output the body temperature T. The first correspondence between Tf and Ts may be established by the processor 110, and stored at a storage location that can be read by the processor 110.

In a possible embodiment, the first correspondence may be a weighted function. For example, the weighted function may be represented as T=w1*Tf+w2*Ts, where w1 and w2 are preset weights, and a sum of w1 and w2 may be 1. Specific weight values are not limited in this application. In this way, after obtaining Tf and Ts, the processor 110 may substitute Tf and Ts into the preset weighted function to obtain a body temperature measurement result (the body temperature T) of the user, and output the body temperature T.

It should be noted that, because Tf can be measured only when the user performs active measurement, and Ts may be obtained through periodic measurement, Tf and Ts may not be aligned. In this case, each time one piece of Ts data is acquired, the Ts value when the user actively measures the body temperature at the most recent time is obtained, and the Ts value is substituted into calculation.

For example, the surface temperature sensor 11 in the smart watch automatically measures the temperature data Ts once every other hour, the user raises the hand to actively measure the body temperature once at 12:00, and the measured temperature of the infrared thermopile sensor 11 is denoted as Tf1. Then, the user raises the hand to measure the body temperature once again at 14:30, and the measured temperature of the infrared thermopile sensor 11 is denoted as Tf2. In this case, when performing body temperature calculation at 14:00, the processor 110 performs weighted processing by using Tf1, to obtain the body temperature T and then output the body temperature T. When the smart watch measures the body temperature at 15:00, Tf2 is used for weighted processing, and the body temperature T is output after being obtained.

In addition, to obtain Ts corresponding to Tf, the surface temperature sensor 13 may also work when the user actively measures the body temperature by raising the hand, and output acquired Ts to the processor 110. This manner helps to obtain a more accurate first correspondence, and helps to improve precision of the body temperature T obtained in this way. Alternatively, if a measurement period of the surface temperature sensor 13 is sufficiently small, for example, once per minute, when the user raises the wrist to perform active measurement, accurate Ts that can correspond to Tf may be obtained.

When the smart watch performs body temperature measurement, a data processing manner during active body temperature measurement and a data processing manner during automatic body temperature measurement may be the same. For example, processing is performed based on the foregoing weighted function. Therefore, when the user actively measures the body temperature by raising the hand, after receiving Tf, if the processor can receive the Ts value corresponding to Tf (corresponding to a same moment), the processor performs weighted calculation by using the Ts value, to obtain the body temperature T of the user, and then output the body temperature T. Alternatively, if there is no Ts value corresponding to Tf, the most recent Ts value may be used for weighted calculation.

The previous example is still used for description. When the user raises the hand for the first time for measurement, the processor 110 may receive Tf1 and the Ts value at 12:00, and then the processor 110 may obtain a single-time measured body temperature T of the user based on Tf1 and the Ts value at 12:00, and output the body temperature T. When the user raises the hand for the second time for active measurement, the processor 110 may receive Tf2, but does not receive the Ts value at this moment. In this case, the processor 110 may use the most recent Ts value, that is, the Ts value measured at 14:00, to participate in weighted calculation, thereby obtaining the body temperature T of the user.

Alternatively, when the smart watch performs body temperature measurement, a data processing manner during active body temperature measurement may be different from a data processing manner during automatic body temperature measurement. For example, when the user raises the wrist to actively measure the body temperature, the processor may directly output the received forehead temperature Tf. When the body temperature of the user is automatically measured in a process in which the user wears the smart watch, the processor may obtain, in the manner shown in FIG. 22, a weighted sum of Tf and Ts after receiving Tf and Ts, to obtain the body temperature T, and output the body temperature T.

Through the weighted processing, the measurement data Ts of the surface temperature sensor 13 can be corrected by using the accurate measurement data Tf of the infrared thermopile sensor 11, to improve precision of the body temperature measurement result.

In another possible embodiment, the first correspondence may be a mapping function. For example, the first correspondence may be represented as Tf=f(Ts). Therefore, after receiving the measured temperature Ts sent by the temperature sensor 13, the processor 110 substitutes Ts into the mapping function, to obtain the temperature value T corresponding to Ts, and output the temperature value T.

In this embodiment of this application, the mapping function may be prestored at a storage location that can be read by the processor 110. The mapping function may be a preset function in the smart watch. Alternatively, the mapping function may be established by the processor 110 based on the received plurality of pieces of measurement data Ts and Tf. For example, in an embodiment, when a quantity of times of active measurement of the body temperature by the user reaches K1, for example, 20, the mapping function may be established by using Tf acquired by the infrared thermopile sensor 11 in the 20 measurement processes and Ts corresponding to Tf. Specifically, when the user actively measures the body temperature, the processor 110 may receive the measured temperatures Tf and Ts, and store Tf and Ts at the preset storage location. In this way, after 20 groups of data of Tf and Ts are accumulated at the preset storage location, the processor 110 may perform curve fitting on a relationship between Tf and Ts, to obtain a mapping function between Tf and Ts, and further store the mapping function at another storage location. The storage location used to store the measurement data may be the same as or different from the storage location used to store the mapping function. This is not limited in this application.

When the mapping function between Tf and Ts is established by the processor 110, after establishing the mapping function, the processor 110 substitutes the received measured temperature Ts into the mapping function, to obtain the body temperature measurement result and output the body temperature measurement result. Before the mapping function is established, the temperature sensor and the processor may be implemented at least in the following manner:

In an embodiment, before the mapping function is established, the surface temperature sensor 13 may measure the temperature Ts based on a preset period or a predetermined moment, and transmit the measured temperature Ts to the processor 110. The processor 110 may receive the measured temperature Ts, store the received measured temperature Ts at the preset storage location, correct Ts based on the foregoing weighted function, to obtain the body temperature T of the user, and output the body temperature T.

In another embodiment, before the mapping function is established, the surface temperature sensor 13 may measure the temperature Ts based on a preset period or a predetermined moment, and transmit the measured temperature Ts to the processor 110. After receiving the measured temperature Ts, the processor 110 stores the measured temperature Ts at the preset storage location. In this case, the processor 110 may temporarily stop calculating the body temperature T. The processor 110 may establish the mapping function between Ts and Tf after K1 groups of measured temperatures are stored. In this way, when receiving Ts again, the processor 110 may correct Ts based on the mapping function, and output corrected Ts, that is, the body temperature T.

In another embodiment, before the mapping function is established, the surface temperature sensor 13 may acquire the temperature data Ts only when the user performs active measurement. That is, the surface temperature sensor 13 also measures the temperature Ts when the infrared thermopile sensor 11 measures the temperature Tf. Therefore, after receiving Ts and Tf, the processor 110 stores the measured temperature Ts at the preset storage location. In this case, the processor 110 may temporarily stop calculating the body temperature T. The processor 110 may establish the mapping function between Ts and Tf after K1 groups of measured temperatures are stored. In this case, the processor 110 may notify the surface temperature sensor 13 to start working. In this way, the surface temperature sensor may measure Ts periodically or in a timing manner, and after receiving Ts, the processor 110 may correct Ts based on the mapping function, and output the body temperature T.

In this embodiment of this application, when automatic body temperature measurement is implemented by using the smart watch, the mapping function between Tf and Ts may be further updated. For example, after the mapping function is established, each time the user actively measures the body temperature for M1 times, for example, 30 times, the mapping function between Tf and Ts is redetermined by using the 30 pieces of Tf data and Ts data corresponding to Tf, to update the mapping function. Then, the processor 110 calculates the body temperature T of the user by using the updated mapping function. The Ts data corresponding to Tf may be determined in the foregoing manner, and details are not described.

In the embodiment shown in FIG. 22, the smart watch corrects the automatically measured temperature by using the temperature actively measured by the user, so that precision of the body temperature measurement result can be improved to some extent.

In another possible implementation scenario, the infrared thermopile sensor 11 is disposed on the front surface of the smart watch, as shown in FIG. 5a, FIG. 5b, FIG. 5c, and FIG. 5d to FIG. 7, and FIG. 14. In addition, the surface temperature sensor 13 is further disposed on the back surface of the smart watch, for example, as shown in FIG. 10, FIG. 13, and FIG. 14. In addition, the smart watch can further obtain the ambient temperature Te, or the ambient temperature sensor 12 is disposed in the smart watch.

In this case, FIG. 23 is a schematic diagram of a body temperature measurement method in this scenario.

In this scenario, the processor 110 may receive three measured temperatures: the forehead temperature Tf, the skin surface temperature Ts, and the ambient temperature Te. In this case, the forehead temperature Tf and the ambient temperature Te may be used to correct Ts.

Therefore, in a possible embodiment, the Tf value corresponding to Ts may be obtained by using the foregoing first correspondence between Tf and Ts, and then, by querying a table, for example, Table 1, a temperature corresponding to the Tf value (the Tf value corresponding to Ts) and the ambient temperature Te is determined, and the temperature is output.

In another possible embodiment, the processor 110 may alternatively first correct the forehead temperature Tf by using the ambient temperature Te, to obtain the corrected forehead temperature, namely, the body temperature Tc on the forehead. For a specific implementation, refer to the embodiment shown in FIG. 21. In this way, the processor 110 may calculate the body temperature T of the user based on a second correspondence and the received measured temperature, which may include, but is not limited to, Ts, and the body temperature T is output.

The second correspondence may be a weighted function. For example, the second correspondence may be represented as T=w3*Tc+w4*Ts, where w3 and w4 are preset weights, and a sum of w3 and w4 may be 1. Specific weight values are not limited in this application. Alternatively, the second correspondence may be a mapping function. For example, the second correspondence may be represented as Tc=f(Ts).

The second correspondence may be prestored at a storage location that can be read by the processor 110.

In addition, when the second correspondence is a mapping function, the mapping function may alternatively be established by the processor 110. For example, the processor 110 establishes a mapping function between Tc and Ts based on K2 groups of measurement data (Tf, Te, and Ts) accumulated at another storage location. Before the mapping function between Tc and Ts is established, the ambient temperature sensor 12 and the surface temperature sensor 13 may work normally, or measure the temperature only when the user performs active measurement. The processor 110 may receive the measurement data, store the measurement data at the preset storage location, and establish the mapping function. Before establishing the mapping function, the processor 110 may not perform an operation of calculating the body temperature T of the user, or the processor 110 may alternatively calculate the body temperature T of the user based on the foregoing weighted function and record the body temperature T. After establishing the mapping function, the processor 110 substitutes the received measured temperature into the mapping function, to calculate the body temperature T of the user and output the body temperature T. Reference may be made herein to the description shown in FIG. 22, and details are not described.

In addition, if the second correspondence is a mapping function, when a quantity of times of active measurement by the user reaches M2, the processor 110 may further update the mapping function between Tc and Ts by using the measurement data stored at the preset storage location, so that the second correspondence is closer to the recent body temperature status of the user. This helps improve measurement precision of the body temperature T.

In another possible implementation scenario, the infrared thermopile sensor 11 is disposed on the front surface of the smart watch, as shown in FIG. 5a, FIG. 5b, FIG. 5c, and FIG. 5d to FIG. 7, and FIG. 14. In addition, the heat flux sensor 14 is further disposed on the back surface of the smart watch. In this case, the heat flux sensor 14 can measure and output the deep tissue temperature Td, as shown in FIG. 12 to FIG. 14.

In this case, FIG. 24 is a schematic diagram of a body temperature measurement method in this scenario.

In this scenario, the processor 110 may receive two measured temperatures: the forehead temperature Tf and the deep tissue temperature Td. Therefore, the processor 110 may calculate the body temperature T of the user by using a third correspondence between the forehead temperature Tf and the deep tissue temperature Td and the received measured temperature (which may include but is not limited to Td), and output the body temperature T of the user.

The third correspondence may be a weighted function. For example, the third correspondence may be represented as T=w5*Tf+w6*Td, where w5 and w6 are preset weights, and a sum of w5 and w6 may be 1. Specific weight values are not limited in this application. Alternatively, the third correspondence may be a mapping function. For example, the third correspondence may be represented as Tf=f(Td).

The third correspondence may be prestored at a storage location that can be read by the processor 110.

In addition, when the third correspondence is a mapping function, the mapping function may alternatively be established by the processor 110. For example, the processor 110 establishes a mapping function between Tf and Td based on K3 groups of measurement data (Tf and Td) accumulated at another storage location. Before the mapping function between Tf and Td is established, the heat flux sensor 14 may work normally or measure the temperature only when the user performs active measurement. The processor 110 may receive the measurement data, store the measurement data at the preset storage location, and establish the mapping function. Before establishing the mapping function, the processor 110 may temporarily stop calculating the body temperature T of the user, or calculate the body temperature T of the user based on the foregoing weighted function and record the body temperature T. After establishing the mapping function, the processor 110 substitutes the received measured temperature into the mapping function, to calculate the body temperature T of the user and output the body temperature T. Reference may be made herein to the description shown in FIG. 22, and details are not described again.

In addition, if the third correspondence is a mapping function, when a quantity of times of active measurement by the user reaches M3, the processor 110 may further update the mapping function between Tf and Td by using the measurement data stored at the preset storage location, so that the third correspondence is closer to the recent body temperature status of the user. This helps improve measurement precision of the body temperature T.

In another possible implementation scenario, the infrared thermopile sensor 11 is disposed on the front surface of the smart watch, as shown in FIG. 5a, FIG. 5b, FIG. 5c, and FIG. 5d to FIG. 7, and FIG. 14. In addition, the heat flux sensor 14 is further disposed on the back surface of the smart watch, for example, as shown in FIG. 12 to FIG. 14. In addition, the smart watch can further obtain the ambient temperature Te, or the ambient temperature sensor 12 is disposed in the smart watch.

In a possible embodiment, the smart watch measures the deep tissue temperature Td of the user by using the heat flux sensor 14. In this case, FIG. 25 is a schematic diagram of a body temperature measurement method in this scenario.

In this scenario, the processor 110 may receive two measured temperatures: the forehead temperature Tf, the ambient temperature Te, and the deep tissue temperature Td. In this case, the deep tissue temperature Td may be corrected by using the forehead temperature Tf and the ambient temperature Te.

Therefore, in a possible embodiment, the Tf value corresponding to Td may be obtained by using the foregoing third correspondence between Tf and Td, and then, by querying a table, for example, Table 1, a temperature corresponding to the Tf value (the Tf value corresponding to Td) and the ambient temperature Te is determined, and the temperature is output.

In another possible embodiment, the forehead temperature Tf may be corrected by using the ambient temperature Te, to obtain the body temperature Tc on the forehead. For a specific implementation, refer to the embodiment shown in FIG. 21. Therefore, the processor 110 may calculate the body temperature T of the user by using a fourth correspondence between the body temperature Tc on the forehead and the deep tissue temperature Td and the received measured temperature (which may include but is not limited to Td), and output the body temperature T of the user.

In another possible embodiment, if the heat flux sensor 14 is configured to measure the heat flux HF, the surface temperature sensor 13 may be further disposed in the smart watch, as shown in FIG. 14. In this case, reference may be made to a schematic diagram of a body temperature measurement method shown in FIG. 26. As shown in FIG. 26, the processor 110 may receive HF and Ts, and calculate the deep tissue temperature Td of the user by using HF and Ts. In this way, the processor 110 corrects the forehead temperature Tf by using the ambient temperature Te, to obtain the body temperature Tc on the forehead. For a specific implementation, refer to the embodiment shown in FIG. 21. Further, the processor 110 may establish the fourth correspondence between Tc and Td based on the received measurement function.

The fourth correspondence may be a weighted function. For example, the fourth correspondence may be represented as T=w7*Tc+w8*Td, where w7 and w8 are preset weights, and a sum of w7 and w8 may be 1. Specific weight values are not limited in this application. Alternatively, the fourth correspondence may be a mapping function. For example, the fourth correspondence may be represented as Tc=f(Td).

The fourth correspondence may be prestored at a storage location that can be read by the processor 110.

In addition, when the fourth correspondence is a mapping function, the mapping function may alternatively be established by the processor 110. For example, the processor 110 establishes the mapping function between Tc and Td based on K4 groups of measurement data (Tf, Te, and Td; or Tf, Te, Ts, and HF) accumulated at another storage location. Before the mapping function between Tc and Td is established, the heat flux sensor 14 may work normally or measure the temperature only when the user performs active measurement. The processor 110 may receive the measurement data, store the measurement data at the preset storage location, and establish the mapping function. Before establishing the mapping function, the processor 110 may temporarily stop calculating the body temperature T of the user, or calculate the body temperature T of the user based on the foregoing weighted function and record the body temperature T. After establishing the mapping function, the processor 110 substitutes the received measured temperature into the mapping function, to calculate the body temperature T of the user and output the body temperature T. Reference may be made herein to the description shown in FIG. 22, and details are not described again.

In addition, if the fourth correspondence is a mapping function, when a quantity of times of active measurement by the user reaches M4, the processor 110 may further update the mapping function between Tc and Td by using the measurement data stored at the preset storage location, so that the fourth correspondence is closer to the recent body temperature status of the user. This helps improve measurement precision of the body temperature T.

In the embodiments shown in FIG. 24 to FIG. 26, the heat flux sensor 14 may work after active measurement for a plurality of times. This reserves time for the heat flux sensor 14 to reach heat balance and facilitates obtaining Td (or HF) with higher precision. In addition, in this embodiment of this application, using FIG. 26 as an example, before a quantity of times of active measurement reaches K4, the processor 110 may correct Td based on the weighted function. This also ensures precision of the body temperature measurement result to some extent.

In addition, in the foregoing embodiments, values of any two of K1 to K4 are the same or different. Values of any two of M1 to M4 are the same or different. This is not limited in this application.

It should be noted that, in this embodiment of this application, the temperature sensor 11 may further measure temperature data of other body parts. For example, the temperature sensor 11 may further be configured to measure a tympanic membrane temperature, an oral temperature, an axillary temperature, and the like. In the embodiments shown in FIG. 20 to FIG. 26, the temperature sensor 11 is configured to measure the forehead temperature. This is a specific embodiment, and should not be construed as a limitation on the technical solutions of this application. In view of this, when the temperature sensor 11 is configured to measure different temperatures, the correspondences adopted in the foregoing embodiments may be different.

For example, in the embodiment shown in FIG. 21, if the user raises the wrist to measure an ear canal temperature (assumed as Tr) at the ear, the temperature sensor 11 transmits the measured ear canal temperature Tr to the processor 110. After receiving the ear canal temperature Tr and the ambient temperature Te, the processor 110 may calculate the tympanic membrane temperature Ta by using a correspondence table among Tr, Te, and Ta (representing the tympanic membrane temperature), and output the tympanic membrane temperature Ta, so that Ta is displayed on the smart watch or the mobile phone.

In another example, in the embodiment shown in FIG. 24, the user may alternatively raise the wrist to measure an axillary temperature (assumed as Tu). When the smart watch performs automatic measurement, after receiving the measured axillary temperature Tu and the deep tissue temperature Td, the processor 110 may calculate the body temperature T of the user based on the correspondence between Tu and Td, and output the body temperature T. For example, the processor 110 may calculate a weighted sum (a sum of weights may be 1) between Tu and Td, and output the weighted sum. In another example, the processor 110 may obtain, based on a preset or pre-established mapping function between Tu and Td, a temperature value corresponding to Td, and output the temperature value corresponding to Td.

In another example, in the embodiment shown in FIG. 25, the user may alternatively raise the wrist to measure the axillary temperature (assumed as Tu). In this way, after receiving the measured axillary temperature Tu and the ambient temperature Te, the processor 110 may use the method shown in FIG. 22, namely, correct Tu by using Te, to obtain a corrected axillary temperature Tv. Therefore, when the smart watch performs automatic measurement, the axillary temperature Tv and the deep tissue temperature Td may be substituted into a weighted function or a mapping function, to calculate the body temperature T of the user and output the body temperature T.

Enumeration is not provided herein.

In this way, the smart watch may determine a currently measured human body part by using a camera. In an embodiment, refer to FIG. 27. FIG. 27 shows a front-surface structure of another smart watch. A camera 16 is disposed on the front surface of the smart watch. The camera 16 may be configured to acquire an image or a video. Specifically, when the user raises the wrist, the camera starts to work; or, when the temperature sensor 11 works, the camera starts to work. The camera and the temperature sensor 11 may communicate directly, or communicate indirectly by using the processor 110. In this way, after the image or video acquired by the camera is output to the processor 110, the processor 110 can perform image recognition on the image or video, to determine a human body part facing the front surface of the smart watch. Further, the processor 110 of the smart watch may determine the body temperature of the user by using a correspondence corresponding to the human body part, and output the body temperature to the display 15 or the mobile phone 200.

It may be understood that the embodiment shown in FIG. 27 is an example. In an actual scenario, the camera 16 may be disposed in any one of the embodiments shown in FIG. 5a, FIG. 5b, FIG. 5c, and FIG. 5d to FIG. 7 and FIG. 10 to FIG. 14.

Alternatively, in the smart watch or the mobile phone, options of different human body parts may be further set. In this way, in an actual scenario, the smart watch may adopt different correspondences based on a selection of the user, to further determine the body temperature of the user. For example, FIG. 28a, FIG. 28b, FIG. 28c, and FIG. 28d are a schematic diagram of another human-computer interaction scenario. As shown in FIG. 28a, FIG. 28b, FIG. 28c, and FIG. 28d, on an interface a, the user may tap a currently connected smart watch, to enter a setting interface (an interface b) of the smart watch. On the interface b, the user may tap a control 219 (the control 219 is used to provide the user with a function of selecting a body temperature measurement part), so that the mobile phone jumps to an interface c. On the interface c, the user is prompted: "When you raise your wrist, the body part facing the front surface of the smart watch is the body temperature measurement part. If you do not perform selection, the forehead is the body temperature measurement part by default. You can modify the body temperature measurement part herein." In addition, a plurality of different body parts and switch controls of the parts, for example, a switch control 220 of the forehead and a switch control 221 of the ear are further displayed on the interface. On the interface c, if the switch control 220 is in an enabled state, a temperature of the forehead part is measured by default when the user raises the wrist. The smart watch may implement the foregoing solution by using correspondences corresponding to the forehead temperature, and obtain the body temperature of the user. In an embodiment, there may be only one switch control that is in the enabled state. In this case, if the user taps the switch control 221, the switch control 221 is enabled, and the switch control 220 is disabled, as shown on an interface d.

In addition, the foregoing two embodiments may be combined. In this case, on the interface c shown in FIG. 28a, FIG. 28b, FIG. 28c, and FIG. 28d, at least one switch control may be in the enabled state. In this case, if the user taps the switch control 221, the switch control 221 is enabled, and the switch control 220 is also enabled. In a specific application scenario, a measurement part is further determined based on the image acquired by the camera 16, and further, which correspondence is adopted to obtain the body temperature T is further determined.

In conclusion, the body temperature measurement method provided in this embodiment of this application provides a wrist body temperature measurement device (the smart watch) that is more comfortable and convenient to operate, and the user may perform active measurement or enable an automatic measurement function based on a requirement of the user, to implement continuous monitoring of the body temperature of the user. This can also provide strong support for female physiological cycle, chronic disease management, circadian rhythm regulation, and the like, can meet different measurement requirements of the user, and have good body temperature measurement experience.

It should be noted that the body temperature measurement method provided in this embodiment of this application is not limited to wrist measurement. In an actual implementation scenario, the electronic device 100 may alternatively be represented as another wearable device. The claims, however, are limited to wrist measurement.

For example, the electronic device 100 may be a smart headset. Refer to a schematic diagram of a structure of a headset shown in FIG. 29. As shown in FIG. 29, the headset is a neck-hanging Bluetooth headset, and the Bluetooth headset includes earbuds and a data line. In an embodiment, the temperature sensor 13 and/or the temperature sensor 14 may be disposed at a location that is on an inner side the data line and that is in contact with the skin of the user. In this way, the temperature sensor 13 and/or the temperature sensor 14 may be configured to measure temperature data on the neck of the skin. Details are not described. In another embodiment, the temperature sensor 11 may be further disposed on a front surface of the earbud, that is, on a side on which an earphone 16 is provided, so that when the user places the earbud into the ear canal, the temperature sensor 11 can measure the tympanic membrane temperature of the user. In another embodiment, the temperature sensor 12 may further be disposed on a side that is on an outer side of the earbud and that is not in contact with the skin of the user. In this way, the temperature sensor 12 is not in contact with the skin of the user, and may be configured to measure the ambient temperature. In addition, the Bluetooth headset may also communicate with the mobile phone of the user by using Bluetooth. In this way, the body temperature of the user measured by the Bluetooth headset may also be displayed on a display interface of the Health APP of the mobile phone, as shown in FIG. 16a, FIG. 16b, FIG. 16c, and FIG. 16d and FIG. 17a, FIG. 17b, FIG. 17c, and FIG. 17d. Details are not described.

For example, the electronic device 100 may alternatively be a smart arm band. The smart arm band may be worn on an arm of the user, and may acquire movement data of the user when started. In this way, at least one of the temperature sensor 13 and the temperature sensor 14 may be disposed on a side that is on an inner side of the smart arm band and that is in contact with the skin of the user. In this way, when the user enables the automatic measurement function, the smart arm band may automatically measure the body temperature of the user in the foregoing manner. In addition, at least one of the temperature sensor 11 and the temperature sensor 12 may also be disposed on a side that is on an outer side of the smart arm band and that is not in contact with the skin of the user. In this way, when the user raises the arm to enable the temperature sensor 11 to be aligned with the forehead or the ear canal of the user, active measurement can be performed. The temperature sensor 12 may be configured to measure the ambient temperature.

In addition, it should be noted that the body temperature measurement method provided in this embodiment of this application may alternatively be implemented by a body temperature measurement system including a plurality of electronic devices. One or more electronic device users may acquire temperature data of a human body, and one electronic device is configured to calculate a body temperature measurement result of the user based on the measured temperature data, and output the body temperature measurement result. For example, in the embodiments shown in FIG. 8, FIG. 16a, FIG. 16b, FIG. 16c, and FIG. 16d to FIG. 19a, FIG. 19b, FIG. 19c, and FIG. 19d, and FIG. 28a, FIG. 28b, FIG. 28c, and FIG. 28d, the smart watch may communicate with the mobile phone. In this case, in these embodiments, one or more smart watches may perform a temperature measurement task, and the mobile phone obtains the body temperature of the user.

The scenario shown in FIG. 26 is used as an example. The infrared thermopile sensor 11, the ambient temperature sensor 12, the surface temperature sensor 13, and the heat flux sensor 14 may be disposed in the smart watch, and are configured to measure temperature data of the user. The processor 110 is disposed in the mobile phone. In this way, the smart watch can measure the temperature data, and send the measured temperature data to the mobile phone. The processor 110 in the mobile phone calculates the body temperature T of the user based on the received measured temperature. Further, the processor 110 may output the body temperature T on a screen of the mobile phone, or the processor 110 may further send the body temperature T to the smart watch, and further display the body temperature T on the display 15 of the smart watch.

In another embodiment of the scenario shown in FIG. 26, the infrared thermopile sensor 11, the ambient temperature sensor 12, the surface temperature sensor 13, and the heat flux sensor 14 may be disposed on a same electronic device, or may be disposed on a plurality of electronic devices. For example, the infrared thermopile sensor 11 and the ambient temperature sensor 12 may be disposed in the mobile phone, and the surface temperature sensor 13 and the heat flux sensor 14 are disposed in the smart watch. In another example, the infrared thermopile sensor 11 and the ambient temperature sensor 12 may be disposed in the smart band, the surface temperature sensor 13 and the heat flux sensor 14 may be disposed in the smart watch, and both the smart band and the smart watch may communicate with the mobile phone, or any two of the smart band, the smart watch, and the mobile phone may directly communicate with each other.

The body temperature measurement method provided in this embodiment of this application is described now with reference to FIG. 30. As shown in FIG. 30, the method includes the following steps.

S3002: Measure a first temperature by using a first temperature sensor, where the first temperature sensor is configured to measure a temperature at a forehead of a user.

S3004: Measure a second temperature by using a second temperature sensor, where the second temperature sensor is configured to measure a temperature at a wrist of the user, and a difference between measurement moments of the first temperature and the second temperature is within preset first duration.

S3006: Display a third temperature on a display, where the third temperature is associated at least with the first temperature.

S3008: Measure a fourth temperature by using the second temperature sensor.

S3010: Display the third temperature on the display when the fourth temperature is the same as the second temperature.

For parts of the method that are not described in detail, refer to the foregoing embodiments. Details are not described herein.

A non-claimed embodiment of this application further provides a computer storage medium, including computer instructions. When run on an electronic device, the computer instructions enable the electronic device to perform the method in any one of the foregoing implementations.

A non-claimed embodiment of this application further provides a computer program product. When run on an electronic device, the computer program product enables the electronic device to perform the method in any one of the foregoing implementations.

In conclusion, the body temperature measurement method, the electronic device, and the computer-readable storage medium that are provided in this application can improve comfort and convenience of a body temperature measurement process and improve measurement precision.

The implementations of this application may be randomly combined to achieve different technical effects.

All or some of embodiments may be implemented through software, hardware, firmware, or any combination thereof. When software is used for implementation, all or some of embodiments may be implemented in a form of a computer program product. The computer program product includes one or more computer instructions. When the computer program instructions are loaded and executed on a computer, all or some of the procedures or functions according to this application are generated. The computer may be a general-purpose computer, a special-purpose computer, a computer network, or another programmable apparatus. The computer instructions may be stored in the computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive Solid-State Drive), or the like.

## Claims

1. A body temperature measurement method, applied to a wrist wearable device (100), wherein the method comprises:
measuring a first temperature by using a first temperature sensor (11), wherein the first temperature sensor (11) is configured to measure a temperature at a forehead of a user, and the first temperature sensor (11) is disposed in the wrist wearable device (100) on a front surface thereof;
measuring a second temperature by using a second temperature sensor (13, 14), wherein the second temperature sensor (13, 14) is configured to measure a temperature at a wrist of the user, the second temperature sensor (13, 14) is disposed in the wrist wearable device (100) on a back surface thereof, and a difference between measurement moments of the first temperature and the second temperature is within preset first duration;
the method being **characterized by**:
displaying a third temperature on a display (15), wherein the third temperature is associated at least with the first temperature;
subsequently measuring a fourth temperature by using the second temperature sensor (13, 14); and
displaying the third temperature on the display (15) when the fourth temperature is the same as the second temperature;
and in that the method further comprises:
obtaining a correspondence between the second temperature and the first temperature when the fourth temperature is different from the second temperature;
obtaining a sixth temperature corresponding to the fourth temperature based on the correspondence; and
displaying the sixth temperature on the display (15).

2. The method according to claim 1, wherein the third temperature is associated with the first temperature and the second temperature.

3. The method according to claim 1, wherein the third temperature is associated with a fifth temperature, and the fifth temperature is obtained by correcting the first temperature by using an ambient temperature.

4. The method according to claim 1, wherein the third temperature is associated with the first temperature, the second temperature and an ambient temperature.

5. The method according to any one of claims 3 to 4, wherein the method further comprises:
measuring the ambient temperature by using a third temperature sensor (12).

6. The method according to any one of claims 1 to 5, wherein the second temperature sensor (14) is configured to measure a deep tissue temperature at the wrist of the user.

7. The method according to any one of claims 1 to 5, wherein the second temperature sensor (13) is configured to measure a skin surface temperature at the wrist of the user.

8. The method according to claim 7, wherein the method further comprises:
measuring a first heat flux by using a heat flux sensor, wherein the heat flux sensor is configured to measure a heat flux between the skin surface temperature and the deep tissue temperature, and a difference between measurement moments of the first heat flux and the second temperature is within preset second duration;
measuring a second heat flux by using the heat flux sensor, wherein a difference between measurement moments of the second heat flux and the fourth temperature is within the second duration; and
displaying the third temperature on the display (15) when the fourth temperature is the same as the second temperature and the first heat flux is the same as the second heat flux.

9. The method according to any one of claims 1 to 8,.
wherein the method further comprises:
establishing the correspondence by using a plurality of first temperatures and a plurality of second temperatures; and
storing the correspondence at a preset storage location.

10. The method according to claim 9, wherein the method further comprises:
updating the correspondence when a quantity of the received first temperatures reaches a preset quantity threshold after the correspondence is established.

11. The method according to any one of claims 1 to 10,
wherein the third temperature is a body temperature measurement result.

12. A wrist wearable device (100), comprising:
a first temperature sensor (11);
a second temperature sensor (13, 14);
one or more processors (110);
one or more memories (121); and
one or more computer programs, wherein the one or more computer programs are stored in the one or more memories (121), the one or more computer programs comprise instructions, and when the instructions are executed by the electronic device (100), the wrist wearable device (100) is enabled to perform the method according to any one of claims 1 to 11.

13. The wrist wearable device (100) according to claim 12, wherein the wrist wearable device (100) further comprises:
a third temperature sensor (12), configured to measure an ambient temperature.

14. The wrist wearable device (100) according to claim 13, wherein when the second temperature sensor (13, 14) is configured to measure the skin surface temperature at the wrist of the user, the wrist wearable device (100) further comprises:
a heat flux sensor, configured to measure a heat flux between the skin surface temperature and the deep tissue temperature.

## Patentansprüche

1. Körpertemperaturmessverfahren, das auf eine am Handgelenk tragbare Vorrichtung (100) angewendet wird, wobei das Verfahren Folgendes umfasst:
Messen einer ersten Temperatur durch Verwenden eines ersten Temperatursensors (11), wobei der erste Temperatursensor (11) dazu ausgelegt ist, eine Temperatur an einer Stirn eines Benutzers zu messen, und der erste Temperatursensor (11) in der am Handgelenk tragbaren Vorrichtung (100) auf einer Vorderseite davon angeordnet ist;
Messen einer zweiten Temperatur durch Verwenden eines zweiten Temperatursensors (13, 14), wobei der zweite Temperatursensor (13, 14) dazu ausgelegt ist, eine Temperatur an einem Handgelenk des Benutzers zu messen, wobei der zweite Temperatursensor (13, 14) in der am Handgelenk tragbaren Vorrichtung (100) auf einer Rückseite davon angeordnet ist und eine Differenz zwischen Messzeitpunkten der ersten Temperatur und der zweiten Temperatur innerhalb einer vorgegebenen ersten Dauer liegt;
wobei das Verfahren **gekennzeichnet ist durch**:
Anzeigen einer dritten Temperatur auf einer Anzeige (15), wobei die dritte Temperatur zumindest mit der ersten Temperatur assoziiert ist;
anschließendes Messen einer vierten Temperatur **durch** Verwenden des zweiten Temperatursensors (13, 14); und
Anzeigen der dritten Temperatur auf der Anzeige (15), wenn die vierte Temperatur gleich wie die zweite Temperatur ist;
und dadurch, dass das Verfahren ferner Folgendes umfasst:
Erhalten einer Entsprechung zwischen der zweiten Temperatur und der ersten Temperatur, wenn sich die vierte Temperatur von der zweiten Temperatur unterscheidet;
Erhalten einer sechsten Temperatur, die der vierten Temperatur entspricht, basierend auf der Entsprechung; und
Anzeigen der sechsten Temperatur auf der Anzeige (15).

2. Verfahren nach Anspruch 1, wobei die dritte Temperatur mit der ersten Temperatur und der zweiten Temperatur assoziiert ist.

3. Verfahren nach Anspruch 1, wobei die dritte Temperatur mit einer fünften Temperatur assoziiert ist und die fünfte Temperatur durch Korrigieren der ersten Temperatur durch Verwenden einer Umgebungstemperatur erhalten wird.

4. Verfahren nach Anspruch 1, wobei die dritte Temperatur mit der ersten Temperatur, der zweiten Temperatur und einer Umgebungstemperatur assoziiert ist.

5. Verfahren nach einem der Ansprüche 3 bis 4, wobei das Verfahren ferner Folgendes umfasst:
Messen der Umgebungstemperatur durch Verwenden eines dritten Temperatursensors (12).

6. Verfahren nach einem der Ansprüche 1 bis 5, wobei der zweite Temperatursensor (14) dazu ausgelegt ist, eine Tiefengewebetemperatur am Handgelenk des Benutzers zu messen.

7. Verfahren nach einem der Ansprüche 1 bis 5, wobei der zweite Temperatursensor (13) dazu ausgelegt ist, eine Hautoberflächentemperatur am Handgelenk des Benutzers zu messen.

8. Verfahren nach Anspruch 7, wobei das Verfahren ferner Folgendes umfasst:
Messen eines ersten Wärmeflusses durch Verwenden eines Wärmeflusssensors, wobei der Wärmeflusssensor dazu ausgelegt ist, einen Wärmefluss zwischen der Hautoberflächentemperatur und der Tiefengewebetemperatur zu messen, und eine Differenz zwischen Messzeitpunkten des ersten Wärmeflusses und der zweiten Temperatur innerhalb einer vorgegebenen zweiten Dauer liegt;
Messen eines zweiten Wärmeflusses durch Verwenden des Wärmeflusssensors, wobei eine Differenz zwischen Messzeitpunkten des zweiten Wärmeflusses und der vierten Temperatur innerhalb einer zweiten Zeitdauer liegt; und
Anzeigen der dritten Temperatur auf der Anzeige (15), wenn die vierte Temperatur gleich wie die zweite Temperatur ist und der erste Wärmefluss gleich wie der zweite Wärmefluss ist.

9. Verfahren nach einem der Ansprüche 1 bis 8,
wobei das Verfahren ferner Folgendes umfasst:
Herstellen der Entsprechung durch Verwenden einer Mehrzahl von ersten Temperaturen und einer Mehrzahl von zweiten Temperaturen; und
Speichern der Entsprechung an einem vorgegebenen Speicherplatz.

10. Verfahren nach Anspruch 9, wobei das Verfahren ferner Folgendes umfasst:
Aktualisieren der Entsprechung, wenn eine Menge der empfangenen ersten Temperaturen nach der Herstellung der Entsprechung einen vorgegebenen Mengenschwellenwert erreicht.

11. Verfahren nach einem der Ansprüche 1 bis 10,
wobei die dritte Temperatur ein Körpertemperaturmessergebnis ist.

12. Am Handgelenk tragbare Vorrichtung (100), umfassend:
einen ersten Temperatursensor (11);
einen zweiten Temperatursensor (13, 14);
einen oder mehrere Prozessoren (110);
einen oder mehrere Speicher (121); und
ein oder mehrere Computerprogramme, wobei das eine oder die mehreren Computerprogramme in dem einen oder den mehreren Speichern (121) gespeichert sind, das eine oder die mehreren Computerprogramme Anweisungen umfassen und, wenn die Anweisungen von der elektronischen Vorrichtung (100) ausgeführt werden, die am Handgelenk tragbare Vorrichtung (100) in der Lage ist, das Verfahren nach einem der Ansprüche 1 bis 11 durchzuführen.

13. Am Handgelenk tragbare Vorrichtung (100) nach Anspruch 12, wobei die am Handgelenk tragbare Vorrichtung (100) ferner Folgendes umfasst:
einen dritten Temperatursensor (12), der zum Messen einer Umgebungstemperatur ausgelegt ist.

14. Am Handgelenk tragbare Vorrichtung (100) nach Anspruch 13, wobei, wenn der zweite Temperatursensor (13, 14) dazu ausgelegt ist, die Hautoberflächentemperatur am Handgelenk des Benutzers zu messen, die am Handgelenk tragbare Vorrichtung (100) ferner Folgendes umfasst:
einen Wärmeflusssensor, der dazu ausgelegt ist, einen Wärmefluss zwischen der Hautoberflächentemperatur und der Tiefengewebetemperatur zu messen.

## Revendications

1. Procédé de mesure de la température corporelle, appliqué à un dispositif portable au poignet (100), le procédé comprenant :
la mesure d'une première température au moyen d'un premier capteur de température (11), le premier capteur de température (11) étant configuré pour mesurer une température au niveau du front d'un utilisateur, et le premier capteur de température (11) étant disposé dans le dispositif portable au poignet (100) sur sa face avant ;
la mesure d'une deuxième température au moyen d'un deuxième capteur de température (13, 14), le deuxième capteur de température (13, 14) étant configuré pour mesurer une température d'un poignet de l'utilisateur, le deuxième capteur de température (13, 14) étant disposé dans le dispositif portable au poignet (100) sur sa face arrière, et la différence entre des instants de mesure de la première température et de la deuxième température s'inscrivant dans une première durée prédéfinie ;
le procédé étant **caractérisé par** :
l'affichage d'une troisième température sur une unité d'affichage (15), la troisième température étant associée au moins à la première température ;
la mesure ultérieure d'une quatrième température au moyen du deuxième capteur de température (13, 14) ; et
l'affichage de la troisième température sur l'unité d'affichage (15) lorsque la quatrième température est identique à la deuxième température ;
et en ce qu'il comprend en outre :
l'obtention d'une correspondance entre la deuxième température et la première température lorsque la quatrième température est différente de la deuxième température ;
l'obtention d'une sixième température correspondant à la quatrième température sur la base de la correspondance ; et
l'affichage de la sixième température sur l'unité d'affichage (15).

2. Procédé selon la revendication 1, dans lequel la troisième température est associée à la première température et à la deuxième température.

3. Procédé selon la revendication 1, dans lequel la troisième température est associée à une cinquième température, et la cinquième température est obtenue par correction de la première température au moyen d'une température ambiante.

4. Procédé selon la revendication 1, dans lequel la troisième température est associée à la première température, à la deuxième température et à une température ambiante.

5. Procédé selon l'une quelconque des revendications 3 à 4, le procédé comprenant en outre :
la mesure de la température ambiante au moyen d'un troisième capteur de température (12).

6. Procédé selon l'une quelconque des revendications 1 à 5, dans lequel le deuxième capteur de température (14) est configuré pour mesurer une température de tissus profonds au niveau du poignet de l'utilisateur.

7. Procédé selon l'une quelconque des revendications 1 à 5, dans lequel le deuxième capteur de température (14) est configuré pour mesurer une température de surface cutanée au niveau du poignet de l'utilisateur.

8. Procédé selon la revendication 7, le procédé comprenant en outre :
la mesure d'un premier flux thermique au moyen d'un capteur de flux thermique, le capteur de flux thermique étant configuré pour mesurer un flux thermique entre la température de surface cutanée et la température de tissus profonds, et la différence entre des instants de mesure du premier flux thermique et de la deuxième température s'inscrivant dans une deuxième durée prédéfinie ;
la mesure d'un deuxième flux thermique au moyen du capteur de flux thermique, la différence entre des instants de mesure du deuxième flux thermique et de la quatrième température s'inscrivant dans la deuxième durée ; et
l'affichage de la troisième température sur l'unité d'affichage (15) lorsque la quatrième température est identique à la deuxième température et que le premier flux thermique est identique au deuxième flux thermique.

9. Procédé selon l'une quelconque des revendications 1 à 8,
le procédé comprenant en outre :
l'établissement de la correspondance au moyen d'une pluralité de premières températures et d'une pluralité de deuxièmes températures ; et
le stockage de la correspondance à un emplacement de stockage prédéfini.

10. Procédé selon la revendication 9, le procédé comprenant en outre :
la mise à jour de la correspondance lorsqu'un nombre des premières températures reçues atteint un seuil de nombre prédéfini après l'établissement de la correspondance.

11. Procédé selon l'une quelconque des revendications 1 à 10,
dans lequel la troisième température est un résultat de mesure de la température corporelle.

12. Dispositif portable au poignet (100), comprenant :
un premier capteur de température (11) ;
un deuxième capteur de température (13, 14) ;
un ou plusieurs processeurs (110) ;
une ou plusieurs mémoires (121) ; et
un ou plusieurs programmes d'ordinateur, le ou les programmes d'ordinateur étant stockés dans la ou les mémoires (121), le ou les programmes d'ordinateur comprenant des instructions qui, lorsqu'elles sont exécutées par le dispositif électronique (100), permettent au dispositif portable au poignet (100) de réaliser le procédé selon l'une quelconque des revendications 1 à 11.

13. Dispositif portable au poignet (100) selon la revendication 12, le dispositif portable au poignet (100) comprenant en outre :
un troisième capteur de température (12), configuré pour mesurer une température ambiante.

14. Dispositif portable au poignet (100) selon la revendication 13, le dispositif portable au poignet (100), lorsque le deuxième capteur de température (13, 14) est configuré pour mesurer la température de surface cutanée au niveau du poignet de l'utilisateur, comprenant en outre :
un capteur de flux thermique, configuré pour mesurer un flux thermique entre la température de surface cutanée et la température de tissus profonds.
